(19) **Europäisches Patentamt / European Patent Office / Office européen des brevets**

(11) **EP 4 712 092 A1**

(12) # EUROPEAN PATENT APPLICATION
published in accordance with Art. 153(4) EPC

(43) Date of publication:
**18.03.2026 Bulletin 2026/12**

(21) Application number: **24803441.5**

(22) Date of filing: **01.05.2024**

(51) International Patent Classification (IPC):
*G16H 20/00* (2018.01)

(52) Cooperative Patent Classification (CPC):
**G16H 20/00**

(86) International application number:
**PCT/JP2024/016833**

(87) International publication number:
**WO 2024/232329 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.05.2023 JP 2023076544**

(71) Applicant: **Provigate Inc.**
**Tokyo 113-0023 (JP)**

(72) Inventors:
• **SEKIMIZU, Koshin**
**Tokyo 113-0033 (JP)**
• **ITO, Narushi**
**Tokyo 113-0033 (JP)**
• **TANIGUCHI, Mariko**
**Tokyo 113-0033 (JP)**
• **MINOURA, Itsushi**
**Tokyo 113-0033 (JP)**

(74) Representative: **Michalski Hüttermann & Partner Patentanwälte mbB**
**Kaistraße 16A**
**40221 Düsseldorf (DE)**

(54) **METHOD FOR ASSISTING BLOOD GLUCOSE CONTROL OF SUBJECT BY USING COMPUTER SYSTEM, AND COMPUTER SYSTEM**

(57) According to an embodiment of the present disclosure, there is provided a method for assisting glycemic control of a subject using a computer system, the method including acquiring a self-set goal of the subject; acquiring GA values of the subject; generating a GA value trend using a plurality of the GA values; outputting the GA value trend to the subject; and outputting the self-set goal of the subject to the subject.

FIG. A1

## Description

TECHNICAL FIELD

The present disclosure relates to a technology for assisting a user in glycemic management based on a GA value using software.

BACKGROUND ART

Diabetes mellitus is one of endocrine diseases characterized by chronically high blood glucose levels (such as high fasting blood glucose levels and high HbAlc values, or the like). Diabetes mellitus is mainly classified into type 1 diabetes, type 2 diabetes, gestational diabetes, diabetes due to pathogenic variants of related genes, and diabetes associated with other diseases and the like. Type 2 diabetes, which has the largest number of patients, is mainly caused by decreased insulin secretion or insulin resistance. Type 1 diabetes is caused by destruction of pancreatic β cells.

Regardless of the type of disease, the basis of treatment is diet therapy and exercise therapy in addition to pharmacotherapy. With these treatments, appropriate glycemic management can be performed and the risk of complications can be reduced. That is, improvement of lifestyle is important. Furthermore, so-called "prediabetes" or "borderline diabetes," having elevated blood glucose levels but not satisfying the diagnostic criteria for diabetes, exists. For persons of this type, improvement of lifestyle is important in order to reduce the risk of onset of diabetes mellitus. As described above, it is considered that improvement of lifestyle including appropriate diet and exercise is effective for glycemic management of diabetes mellitus and prevention thereof.

Treatment of the chronic disease of diabetes mellitus is generally considered to be difficult. Reasons include that it progresses without subjective symptoms; it is difficult to maintain treatment motivation; complete cure is difficult and treatment must be continued for a lifetime; and improvement of lifestyle as well as drug therapy is essential. These are considered to make self-management required for treatment difficult (NPL 1).

In fact, there is a report that it takes 18 to 254 days, with a median of 66 days, for a new habit to become automatic (NPL 2). Based on this, behavioral change requires two to several months. In general, some kind of system is required to maintain motivation for such a period of time.

Typical examples of biomarkers for glycemic control are blood glucose levels, HbAlc values, and GA values. However, fasting blood glucose levels can hardly be considered to reflect lifestyle. HbAlc is considered to reflect blood glucose levels over the past two to three months. However, it is difficult to maintain motivation for self-management for such a long period of time. In other words, it is too long as feedback for behavioral change. On the other hand, albumin has a blood half-life of about 17 days. Accordingly, a glycoalbumin level (GA value) reflects blood glucose levels over a relatively short term, namely, the past one to three weeks.

CITATION LIST

NON PATENT LITERATURE

NPL 1: American Diabetes Association, Diagnosis and classification of diabetes mellitus, Diabetes Care. 2014; 37 Suppl 1:S81-90. doi: 10.2337/dc14-S081., https://doi.org/10.2337/dc14-s081
NPL 2: Lally P, van Jaarsveld C, Potts H, Wardle J. How are habits formed: Modelling habit formation in the real world. Eur J Soc Psychol. 2009; 40(6):998-1009. doi:10.1002/ejsp.674

SUMMARY OF INVENTION

It is desired to utilize GA values for improvement of lifestyle and behavioral change. Accordingly, an object of the present disclosure is to provide a technology for assisting diabetes patients and the like in effectively changing and maintaining their own behaviors using GA values.

Based on intensive efforts and extensive clinical studies, the inventors have found that when a user checks weekly changes in a GA value, which is their own biomarker, and recognizes their own "goal" at a similar frequency, the GA value decreases over a long period of time.

According to some embodiments of the present disclosure, there are provided a method, a computer system, and software for assisting a subject who performs or intends to perform glycemic management, improvement of lifestyle, and/or behavioral change based on a GA value. The present disclosure provides methods for diabetes management, glycemic management, glycemic control, blood glucose level control, glycemic control management, glycemic regulation, management of blood glucose levels, adjustment of blood glucose levels, diabetes treatment, regulation of glucose metabolism, and the like.

In some embodiments, a goal of a subject is acquired. In some embodiments, a GA value of the subject is acquired. In some embodiments, the GA value is output to the subject. In some embodiments, the goal of the subject is output to the subject.

In one embodiment, the present disclosure may cause a computer system to execute the methods. In some embodiments, the computer system may include a processor; a communication unit connected to the processor and capable of communicating with an electronic terminal accessible by a subject; and a memory connected to the processor. In some embodiments, a computer is caused to acquire a goal of the subject. In some embodiments, the computer is caused to acquire a GA value of the subject. In some embodiments, the computer is caused to output the GA value to a terminal of the subject. In some embodiments, the computer is caused to output the goal of the subject to the terminal of the subject.

ADVANTAGEOUS EFFECTS OF INVENTION

A general life cycle in the modem era is established using the week as a unit of time. Accordingly, it is consistent with the life cycle that the user recognizes their own GA value and their own goal on a weekly basis. Therefore, it is effective in, for example, increasing awareness of lifestyle, initiating behavioral change voluntarily to improve lifestyle, and maintaining motivation.

It is to be noted that the above effects are not necessarily limiting, and together with or in place of the above effects, any of the effects described in this specification or other effects that can be understood from this specification may be achieved.

BRIEF DESCRIPTION OF DRAWINGS

Fig. A1 is a sequence chart of a process of a system and communication between the system and a subject according to an embodiment.

Fig. A2 is a sequence chart of a process of a system and communication between the system and a subject according to an embodiment.

Fig. A3 is a sequence chart of a process of a system and communication between the system and a subject according to an embodiment.

Fig. A4 is a sequence chart of a process of a system and communication between the system and a subject according to an embodiment.

Fig. A5 is a sequence chart of a process of a system and communication between the system and a subject according to an embodiment.

Fig. A6 is a sequence chart of a process of a system and communication between the system and a subject according to an embodiment.

Fig. A7 is a sequence chart of a process of a system and communication between the system and a subject according to an embodiment.

Fig. A8 is a sequence chart of a process of a system and communication between the system and a subject according to an embodiment.

Fig. B1 illustrates graphs displaying GA value trends according to an example.

Fig. B2 illustrates examples of GA value trends and messages according to an example.

Fig. C1 illustrates a display screen of an electronic terminal of a subject according to an example.

Fig. C2 illustrates a display screen of the electronic terminal of the subject according to an example.

Fig. C3 illustrates a display screen of the electronic terminal of the subject according to an example.

Fig. C4 illustrates a display screen of the electronic terminal of the subject according to an example.

Fig. D1 is a graph representing changes in GA value obtained in an example.

Fig. D2 is a graph representing changes in HbAlc value obtained in an example.

Fig. D3 is a graph representing changes in body weight obtained in an example.

Fig. D4 is a graph representing changes in BMI obtained in an example.

Fig. D5 is a graph representing changes in abdominal circumference obtained in an example.

Fig. E1 illustrates a network configuration including a system and a subject terminal according to an embodiment.

Fig. E2 is a block diagram representing functional components of a system according to an embodiment.

Fig. F1 is a flowchart of a method according to an embodiment.

Fig. G1 is a graph representing a relationship between the number of characters input to the system and a decrease in GA value obtained in an example.

Fig. G2 is a flowchart of generation of output information according to an embodiment.

DESCRIPTION OF EMBODIMENTS

The following description describes numerous specific details in order to provide a more complete understanding of the present invention. However, it will be apparent to a person skilled in the art that the present invention may be implemented with one or more of these specific details. In other examples, features and procedures well known to a person skilled in the art are not described in order to avoid obscuring the present invention.

Some embodiments and examples will be described with reference to the accompanying drawings. In sequence diagrams, arrows represent transmission of information or an order of processes (steps). However, no order is specified between processes not defined by arrows, as long as there is no inconsistency. The nature of the arrows is also applied to other sequence diagrams. In addition, when a plurality of embodiments and drawings have similar functions, configurations, or steps, such functions, configurations, or steps may be omitted from one of them. These are omitted on the premise of being understandable to a person skilled in the art. This does not necessarily mean that the functions, configurations, or steps in the plurality of embodiments and drawings are the same.

Unless defined otherwise, all technical and scientific terms used herein have the same meaning as commonly understood by a person skilled in the technical field to which the present invention pertains.

As used herein, the terms "including" and "comprising" are intended to mean that configurations and methods include the enumerated elements but do not exclude others. Embodiments defined by each of these transitional terms fall within the scope of the present invention. Accordingly, methods, compositions, and configurations may "include" additional steps and components.

As used herein, the term "subject" refers to a person who uses a system according to any embodiment of the present disclosure or, in a computer-implemented method according to any embodiment of the present disclosure, performs their own glycemic management with assistance from a computer system. That is, the subject provides, or authorizes a third party to provide, a GA value of their own body fluid to the system. The system acquires the GA value and assists glycemic management of the subject. The subject may be a diabetes patient. For example, the diabetes patient may be a type 2 diabetes patient. The subject may be a non-diabetes patient. A non-diabetes patient refers to people in general who have not been diagnosed as having diabetes mellitus. The subject may be a prediabetic individual and/or a healthy individual.

On the other hand, as used herein, the term "user" refers to a person who uses a computer system according to any embodiment of the present disclosure or uses the computer system in a method implemented by the computer system according to any embodiment of the present disclosure. The "user" includes the subject and further includes a person who can lawfully use the system, method, or computer system in relation to the user. For example, the "user" includes a healthcare professional, a counselor, and the like. The "user" includes a person who is currently using, should use in the future, is planned to use, or has used the system of the present disclosure.

As used herein, the term "computer system" generally refers to equipment programmed to automatically execute a sequence of arithmetic or logical operations, including hardware composed of electronic equipment or components, an operating system, software, and peripheral equipment as needed. The term "computer system" is used interchangeably with "computer" and "system". The "computer system" may be a physically single computer, a plurality of computers connected via a network, or a part thereof. The "computer system" may be a plurality of connected computers, and parts of respective computers are configured by being connected among a plurality of computers. For example, the "computer system" may be configured to include a computer center capable of assisting glycemic management of a large number of users, an electronic terminal of a certain user, and application software installed in and functioning on the electronic terminal. Alternatively, the "computer system" may be configured to include an electronic terminal of a certain user and application software installed in and functioning on the electronic terminal. For example, application software serving as a user interface is installed in an electronic terminal of a user, and various computations may be performed at a computer center communicable with the electronic terminal. A memory or storage that stores various data may be a part of the computer system, or may be disposed so as to be communicable with a computer system external to the computer system.

Each step of the methods described herein should be interpreted as being executed by a computer system or a part thereof (for example, a CPU), unless the subject thereof is specifically specified. Further, even when the subject of each step is specified, if there is no inconsistency in being executed by the computer system or a part thereof, the step may also be read as being executed by the computer system or a part thereof (for example, a CPU).

The system may be configured to authorize only a subject or others with legitimate rights to access the GA value and GA value trend of the subject, input information from the subject, and output information to the subject and to block others without legitimate rights from accessing them. While embodiments or examples of the present disclosure mainly describe input and output of information performed between one subject and the system, the present invention is not limited to these embodiments or examples. The methods, systems, and the like of the present disclosure may be configured to be capable of providing services to a plurality or a large number of subjects. With reference to input information from a large number of subjects, processing and optimization of the information, secondary processed data processing, output information to a large number of subjects, and the like, information processing and optimization, secondary processed data processing, generation of output information, and the like relating to each subject may be executed.

<Measurement of GA Value>

The term "GA value" generally refers to an amount obtained by dividing the amount of glycoalbumin in the body fluid of a subject by the total amount of albumin in the body fluid of the subject. Examples of methods for measuring a GA value include, without limitation, an enzymatic method, a high-performance liquid chromatography method, a mass spectrometry method, an isotope dilution mass spectrometry method, and the like.

In some embodiments, an enzymatic method may be used as a clinical test method. Glycoalbumin may be decomposed to amino acids with a protease, a ketoamine oxidase that reacts only with glycated amino acids among the amino acids may be allowed to act, and $H_2O_2$ generated by the action may be measured to obtain the glycoalbumin concentration. Albumin concentration may be obtained by reacting albumin with a bromocresol purple (BCP) reagent and measuring a change in the absorbance of a blue complex.

Generally, a GA value is defined as the glycation ratio of albumin. For example, the GA value can be calculated as [glycoalbumin concentration/albumin concentration] $\times$ 100 (%). Alternatively, a relational expression between an analytical value of a glycoalbumin standard specimen and a GA value linked to the standard specimen, and a conversion expression (conversion from mmol/mol to %) may be prepared in advance. The relational expression may be obtained by linear regression of the two values. The GA value can be obtained by measuring the total albumin concentration and the glycoalbumin concentration in a specimen and based on the above relational expression. More specifically, the GA value may be defined by another formula, and the definition may be changed in detail in the future.

In some embodiments, a glycated protein sensor including an immobilized protease, an immobilized ketoamine oxidase, and a hydrogen peroxide detection section may be used to measure the concentration of glycoalbumin.

For example, by using such a sensor, the GA value in tear fluid or saliva can be measured non-invasively. Alternatively, the GA value of a small amount of blood such as puncture blood, or the like can be measured in a minimally invasive manner. Accordingly, it is possible to measure GA values without imposing physical or mental stress on an examinee or while minimizing a burden of such stress on the examinee.

<Weekly GA Value>

The term "weekly GA value" as used herein refers to a GA value of a body fluid acquired at intervals of approximately one week. Alternatively, the interval of acquisition of a GA value need not be constant. In some embodiments, a GA value may be acquired at a frequency of once in a predetermined cycle (for example, one week). The interval, the cycle, or the frequency may be adjusted or varied according to convenience of the subject. For example, it may not be possible to collect body fluid on the same day of the week every week, that is, days of the week on which body fluid is collected may differ among a plurality of weeks. For example, the cycle need not be one week. There are subjects whose lifestyle is not based on a weekly cycle. For example, a cycle of five days, six days, eight days, nine days, or the like may be adopted. Lifestyle changes may occur every several weeks, every several months, or the like. Each time, settings of the interval, the cycle, or the frequency may be changed.

For example, a GA value may be measured at a frequency of substantially once per month, twice per month, or higher. For example, the frequency may be less than or equal to a value such as 20 times per month, 10 times per month, 8 times per month, or once per month, or may be less than the value. For example, the frequency may be substantially twice per month, three times per month, or four times per month, or may be 0.5 times per week or once per week.

In some embodiments, the frequency may be substantially once every 17 days. In some embodiments, the frequency may be about once every two weeks.

GA values may be measured at intervals of an average of 7 days, an average of 10 days, an average of 14 days, or more. GA values may be measured at intervals of an average of 21 days, an average of 24 days, an average of 28 days, or less.

The system may notify the subject of a timing at which body fluid is to be next collected. For collections after the first time, notification may be performed at regular or irregular intervals. For example, a notification (reminder) of body fluid collection may be displayed on an electronic terminal accessible by the subject. The notification of body fluid collection may be displayed on the subject's terminal at various timings. A date of body fluid collection may be programmed to be always displayed on the subject's terminal when the terminal is on. A date of next body fluid collection may be displayed on the subject's terminal when a predetermined application is launched or being used by the subject. A date of next body fluid collection may be displayed on the subject's terminal in response to a predetermined action (recognizable by a sensor such as a sound, acceleration, or light sensor, or the like) by the subject toward the terminal, which acts as a trigger. When a predetermined date approaches (one day before or on the day), the subject may be notified as a separate reminder. When a scheduled date of body fluid collection has passed, the subject may be notified as a separate reminder to promptly collect body fluid.

As used herein, the expressions "time of a GA value", "measurement time of a GA value", and "time at which a GA value is to be acquired/is acquired" or similar time-related expressions are based not on a time at which the GA value is measured but on a time at which a body fluid or specimen containing glycoalbumin and albumin from which the GA value is derived is acquired, unless otherwise defined.

<Acquisition of GA Value>

In some embodiments, a subject may acquire their own body fluid (for example, blood, saliva, or the like) and cause the body fluid to be transported, by a predetermined method, to a laboratory having equipment for measuring a GA value. The transportation may be by mail. The laboratory, which has received the body fluid of the subject, may obtain the GA value in the body fluid of the subject. In some embodiments, the laboratory may transmit the obtained GA value to the system. Thus, the system can acquire the GA value. In some embodiments, the laboratory may store the acquired GA value in a predetermined storage, and the system may access the storage to acquire the GA value.

In some embodiments, a subject may visit a medical institution such as a hospital, a pharmacy, a school, a convenience store, a fitness club, a government office, a public hall, or any other public facility, or the like (hereinafter sometimes simply referred to as a hospital). For example, the subject may visit a medical institution to submit a specimen or for measurement. The medical institution may acquire body fluid of the subject and perform measurement of a GA value in an internal or affiliated hospital. GA value measuring equipment may be, for example, point-of-care testing (POCT) equipment (hereinafter sometimes simply referred to as POCT equipment) or in vitro diagnostic (IVD) medical equipment. The medical institution may acquire body fluid of the subject and transport the body fluid to an external laboratory to have the laboratory measure a GA value. In some embodiments, the medical institution may transmit the obtained GA value to the system. Thus, the system can acquire the GA value. In some embodiments, the medical institution may store the acquired GA value in a predetermined storage, and the system may access the storage to acquire the GA value.

In some embodiments, a subject may have GA value measuring equipment at home or a residence. The GA value measuring equipment may be, for example, POCT equipment, in vitro diagnostic (IVD) medical equipment, home medical equipment, a self-measuring instrument, or a self-monitoring apparatus. The subject may acquire their own body fluid or have another person acquire the body fluid of the subject, and may measure a GA value with the measuring equipment. In some embodiments, the self-monitoring apparatus may transmit the obtained GA value to the system. Thus, the system can acquire the GA value. In some embodiments, the self-monitoring apparatus may store the acquired GA value in a predetermined storage, and the system may access the storage to acquire the GA value.

<Trend of GA Values>

As used herein, the term "trend of GA values" or "GA value trend" generally refers to information including a direction and/or a fashion of changes in (at least two different GA values in chronological order. The "trend of GA values" may include information related to a change in at least two different GA values in chronological order, that is, a change such as an increase (up, raise, increase, or the like)/a decrease (down, fall, decrease, or the like)/no change (flat, unchanged, constant, or the like) of a later GA value relative to a previous GA value.

In some embodiments, the "GA value trend" may be provided as a graph (for example, a line graph). A subject can recognize the trend of their own GA values at a glance. The format of the "GA value trend" is not limited to the above and may be selected, for example without limitation, from text, images, videos, and the like.

In some embodiments, the "GA value trend" may be defined using a set of GA values measured twice consecutively. In some embodiments, when the n-th GA value is greater than/less than/equal to the (n-1)-th GA value, it is defined that the GA value has increased/decreased/been flat, respectively.

In some embodiments, the "GA value trend" may be expressed using GA values obtained by three consecutive measurements {(n-2)-th, (n-1)-th, and n-th}, by using the movements of two GA values. In some embodiments, the "GA value trend" may be expressed using two changes, namely, a change in GA value from the (n-2)-th measurement to the (n-1)-th measurement (consecutive change 1) and a change in GA value from the (n-1)-th measurement to the n-th measurement (consecutive change 2). In some embodiments, a pattern group made up of $3 \times 3 = 9$ patterns of consecutive change 1 = {increase, decrease, flat} and consecutive change 2 = {increase, decrease, flat} may be defined.

In some embodiments, the "GA value trend" may be expressed using a plurality of GA values that do not include two consecutive values, that is, a plurality of GA values that include at least one value that is measured discontinuously. In some embodiments, the "GA value trend" may be expressed using the difference (absolute change) = {increase, decrease, flat} of the n-th GA value with respect to the initial GA value.

Here, the "GA value" at each point need not be a single GA value, and other definitions are possible. For example, each point may be defined by a statistical value (such as a mean value) of GA values acquired over a plurality of weeks, and three or more statistical "GA values" may be displayed in a time series.

In some embodiments, the "GA value trend" may be expressed using a plurality of definitions of "trends" or "changes". In some embodiments, the "GA value trend" may be expressed by combining "consecutive change 1", "consecutive change 2", and "absolute change". Specific examples will be described in Examples.

In some embodiments, the "n-th GA value" described above may be the latest GA value.

Typically, a "GA value trend" is formed based on a plurality of GA values. However, when there is only one GA value available, the GA value trend may be formed only with that one GA value. Only available GA values may be output, for example, immediately after the start of use of the system, when data is lost, when graph display does not function, and the like.

In some embodiments, the "GA value trend" may include, at least in part, one or more GA values that are possible in the future, that is, a trend of future GA values. The future GA values may be predicted based on any or a combination of the latest GA value, the past GA values, behavior-related information, or the like.

<Output of GA Value Trend>

In some embodiments, when a new GA value is acquired, the GA value may be promptly output to a subject. The subject can learn the transmitted latest GA value through an electronic terminal. In some embodiments, when a new GA value is acquired, a GA value trend including the GA value may be output to a subject. The subject can learn the transmitted GA value trend through the electronic terminal.

In some embodiments, a GA value may be provided so as to be accessible by a subject even at a point in time other than the timing at which the GA value is acquired. For example, the subject may start a predetermined application or issue a command using a terminal. Based on the input, the system may display the acquired GA value and/or GA value trend on the terminal of the subject.

<Behavior-Related Information>

As used herein, the term "behavior" generally refers to lifestyles, activities, or acts related to so-called lifestyle-related diseases including diabetes mellitus. The "behavior" can affect lifestyle-related diseases such as blood glucose levels, HbAlc values, GA values, or values or data corresponding or related thereto (for example, CGM data).

In some embodiments, the "behavior" includes exercise, diet, and/or medication. All of exercise, diet, and medication affect blood glucose levels, HbAlc values, and GA values.

In some embodiments, information related to the "behavior" (also referred to as "behavior-related information") may be input from an electronic terminal. Examples of formats of behavior-related information include, without limitation, text information, voice information, handwriting information, image information, position information, communication information, biometrics, and the like. In some embodiments, the system may provide a subject with an option to input behavior-related information to the system via an electronic terminal. In some embodiments, the system may request a subject to input behavior-related information to the system via an electronic terminal.

In some embodiments, a subject may input selected answers to multiple-choice questions about exercise, diet, and medication. In some embodiments, a subject may input sentences in response to sentence open-ended questions. Questions provide the subject with an opportunity to input answers to the system by the subject themselves. The act of inputting as well as performing a self-evaluation regarding the subject's own behavior is considered to help build awareness for blood glucose.

In some embodiments, the "behavior-related information" may be configured such that a subject can select one or more from several preset choices on a terminal. For example, choices may be displayed as push buttons on a touch panel. For example, a question with three choices such as "well done", "average", and "failed" may be provided.

In some embodiments, the "behavior-related information" may be input in natural language. For example, behavior may be input in the form of a diary. A subject can describe, concisely or in detail with their own words, behaviors performed on that day, that week, or during a predetermined period of time. Through the diary, the subject may also raise questions.

The system may prompt the subject to input behavior-related information at a predetermined frequency (for example, daily). For example, behavior-related information may be input as part of the subject's "diary". Input of daily exercise, diet, and medication is considered to be useful for building the subject's awareness.

In some embodiments, the system may provide an option for a subject to input an evaluation (self-evaluation) regarding behaviors performed by the subject. Thus, the subject can input behavior-related information by themselves. Input through the three-choice question as described above and input in natural language, and the like are examples thereof. By inputting by themselves, the subject can recognize their own behavior and can change the behavior voluntarily rather than compulsively. Alternatively, the probability thereof increases. Voluntary behavioral change may contribute to high sustainability and effective glycemic control.

In some embodiments, behavior-related information may be input by a non-subject. For example, a caregiver (including, for example without limitation, a spouse, family, friends, nurses, physicians, attorneys, and others) may input each piece of information. The caregiver may understand the intention of the subject. The caregiver may have legitimate rights.

<Goal>

As used herein, the term "goal" generally refers to biological information and/or behavior that is related to lifestyle-related diseases and that a subject is to achieve.

Examples of markers as goals include, without limitation, GA values, HbAlc values, body weight, BMI, and the like. The subject may set a value of a marker to be achieved or to be maintained at or during a predetermined timing or period of time in the future. In some embodiments, a marker as a goal is a GA value. Examples of behaviors as goals include, without limitation, diet, exercise, medication, and the like. The subject may set the content of diet, exercise, and/or medication to be implemented at or during a predetermined timing or period of time in the future. In some embodiments, behaviors as goals are diet, exercise, and medication.

Examples of goals related to "exercise" include, without limitation, type of exercise: walking, swimming, strolling, stair climbing, squats, and the like; time zone: after meals, early morning, and the like; and amount: frequency, intensity, number of steps, time, and the like. Examples of goals related to "diet" include, without limitation, things to eat/not to eat: refraining from consuming sweet drinks such as juice, stopping snacking, refraining from eating sweets as snacks, trying to consume foods containing fiber, such as vegetables, reducing carbohydrates such as white rice and bread, and reducing alcohol intake; amount of diet; frequency of diet; and amounts or balance of lipids, carbohydrates, and salt (FC balance, FCS balance), and the like. Examples of goals related to "medication" include, without limitation, taking prescribed drugs as directed and avoiding missed doses, and the like.

Examples of timing at or by which a goal has been achieved or a period of time during which a goal is achieved or maintained once or multiple times include, without limitation, next week, a plurality of weeks, timing of next HbA 1c measurement, and the like.

<Setting/Changing of Goal>

In some embodiments, the "goal" may be set by the subject, voluntarily, spontaneously, proactively, autonomously, intrinsically, or actively. As used herein, the adverbial expressions at the time of setting a goal, "by themselves", "voluntarily", "spontaneously", "proactively", "autonomously", "intrinsically", and "actively" have substantially the same meaning or at least partially overlapping meanings, unless otherwise defined, and are used interchangeably.

In some embodiments, a subject may determine a goal voluntarily with support from an assistant such as a physician, a nurse, a therapeutic instructor, a psychological counselor, a registered dietitian, or a mentor or from the system, or alone. Voluntary goal setting can enhance self-efficacy.

In some embodiments, a goal may be set together with support from an assistant (such as a supporter or a consultant) or the system. A so-called joint goal may be set. When a goal desired by a subject is determined to be unrealistic, an assistant or the system may propose an achievable goal. When a goal desired by the subject is determined to be unrealistic, difficult to implement, or ineffective, the system may propose a more realistic, more feasible, or more effective goal to the subject. For example, when the subject desires to set a goal of an extreme decrease in GA value, a goal that can be reached or is easy to reach in a relatively short period of time may be proposed. As a specific nonlimiting example, when the subject desires to achieve a goal of a 2% decrease in GA value in one week, it may be proposed that a 0.5% decrease should be the initial goal. Accordingly, the subject becomes more likely to achieve the goal, and the subject can feel satisfaction in achieving the goal while avoiding rebound due to excessive dietary restriction or a hypoglycemia risk.

For example, an optimal goal for the subject may be set by motivational interviewing (MI). In particular, it is considered effective that an initial goal is set using motivational interviewing. However, motivational interviewing may be performed for goal setting at any point in time. In some embodiments, motivational interviewing may be conducted via human counseling. In some embodiments, motivational interviewing may be performed by the system.

In some embodiments, a "goal" is proposed to a subject by the system (a proposed goal is output), and the subject may refer to the proposed "goal" (proposed goal). The subject recognizes the goal proposed by the system, but is free to follow it, use it as a reference, or ignore it.

The subject can determine the next goal by themselves or input it to the system. Rather than the system or a third party setting the subject's goal, the subject voluntarily setting their own goal is considered effective for behavioral change.

A "goal" may be changed periodically or non-periodically. The system may provide the subject with an opportunity to set a new goal. For example, the subject may be given an opportunity to change a goal every week or each time a latest GA value is output from the system. The subject may change the goal at a change opportunity. The subject may maintain the previous goal without changing it. Regardless of whether the previous goal is changed or not, it is considered effective that the subject determines the next goal by the subject's own will.

The system may provide the "goal" set by the subject themselves to the subject through an electronic terminal used by the subject. The "goal" may be displayed simultaneously with or on the same screen as the GA value or the "GA value trend". The latest or updated goal may be provided to the subject. The subject can recognize their latest GA value or "GA value trend" and their current "goal". Recognition of a change in a glycemic biomarker, namely, a GA value, and awareness building of behaviors to be performed by the subject may be linked in the conscious or subconscious mind of the subject. This can positively affect behavioral change or improvement of lifestyle. As described above, one preferable method that enables the subject to substantially simultaneously recognize the self-set goal of the subject and the GA value is to use an app installed on an electronic terminal that the subject uses daily. It is effective in modern society to assist glycemic management using electronic equipment rather than using paper media such as planners and books or oral communication.

The "goal" and/or the "GA value trend" can be displayed on the terminal of the subject at various timings. The "goal" and/or the "GA value trend" may be programmed to be always displayed on the subject's terminal when the terminal is on. The "goal" and/or the "GA value trend" may be displayed on the subject's terminal when a predetermined application is launched or being used by the subject. The "goal" and/or the "GA value trend" may be displayed on the subject's terminal in response to a predetermined action (recognizable by a sensor such as a sound, acceleration, or light sensor, and the like) by the subject toward the terminal, which acts as a trigger.

For example, the "goal" and/or the "GA value trend" may be displayed on a diary input screen. The subject can recognize the "goal" and/or the "GA value trend" when reflecting on their own behavior of the day. Accordingly, the subject can recognize the behavior performed by the subject (diary), actions to be taken and/or a target GA value ("goal"), and a GA value reflecting latest behavior improvement from the previous behavior ("GA value trend"). That is, awareness building of behaviors to be performed by the subject and actions actually taken, and a GA value change as an evaluation thereof, may be linked in the conscious or subconscious mind of the subject. This can positively affect behavioral change or improvement of lifestyle.

<Provision of Beneficial Information>

As used herein, the term "beneficial information" generally refers to information output from the system to the subject, other than GA values, GA value trends, and goals. The "beneficial information" is a type of "output information". The "beneficial information" may be any information that can be "beneficial" to the subject, and it does not necessarily matter whether the "beneficial information" is information regarded objectively as beneficial for glycemic management, or is subjectively "beneficial" to the subject or "beneficial" based on past, present, or future theories or scientific findings.

The "beneficial information" can be generated or provided to the subject in various forms. In some embodiments, beneficial information includes visual information. Examples of visual information include still images, videos, and the like. For example, a video clip may include education-based information (also referred to as educational information or an educational video). In some embodiments, beneficial information includes text information. The text information may be displayed as characters on a display of an electronic terminal or may be converted into voice and provided to the subject as voice information from the electronic terminal.

The beneficial information may include knowledge related to lifestyle (also referred to as "educational information"). Generally, subjects lack knowledge effective for lifestyle-related diseases (also referred to as "correct knowledge". The term "correct" does not guarantee that the modified content has been scientifically proven to be "correct", that future theories or practices will not change, or that all skilled persons, subjects, and healthcare professionals recognize the content as "correct"). For example, in some cases, a subject does not understand the content of diet or exercise regarded as correct for the subject. In some embodiments, the system may provide "correct knowledge" determined to be effective for a subject at that point in time, based on various kinds of input information. For example, the "correct knowledge" may be provided in a method, format, or style that is easy for the subject to accept. In some embodiments, the system may provide information that a subject needs or desires in a state in which the information is always available. The subject can collect information by themselves using the system and perform self-management.

<Chatbot>

The system may provide a chatbot (ChatterBot). In some embodiments, a chatbot may be included in the beneficial information. In some embodiments, a chatbot may be categorized separately from the beneficial information. The chatbot may be configured to be able to exchange messages with the subject and/or to communicate with the subject. For example, the chatbot may output a message (system message) to the subject and/or acquire a message (user message) from the subject. The chatbot may be configured to answer questions from the subject. The chatbot may approach the subject independently of a request from the subject, or spontaneously.

For example, the chatbot may output information for psychologically supporting the subject to the terminal of the subject as a system message. The system message may include content related to glycemic management, content not directly or indirectly related thereto, or both. The system may be configured to generate system messages or communicate with the subject in order to encourage the subject to continue using the system, maintain a relationship with the subject, improve the relationship with the subject, and/or facilitate rapport with the subject.

"Messages" can be output at various timings. For example, when no question ("chatbot" input) is received from the subject during a predetermined period of time (for example, one week, two weeks, three weeks, two GA value outputs, three GA value outputs, or the like), or when there is no "diary" input from the subject, there is a lot of missing information in "diary" input, or the amount of missing information has increased during the predetermined period of time, a message may be programmed to be posed to the subject.

In some embodiments, the subject may be notified that there is no or little input information. In some embodiments, when it is determined that there is no or little input information, the system may avoid directly referring to the matter.

In some embodiments, for example, consistency and discrepancies between input information from a subject, such as a "diary", a "review", and a "message" (user message), and a GA value trend, and/or among a plurality of pieces of input information may be analyzed.

For example, when a "GA value trend" is deteriorating whereas diet information in a "diary" includes an input that diet control was successful, it may be determined that there is a discrepancy between the two. In that case, the system may look for other causes of deterioration of the GA value trend. For example, without limitation, the possible causes can be assumed to be the subject's lack of understanding, missed or changed medications, poor physical condition, and input mistakes, and the like.

In some embodiments, the subject may be notified that there is a possible discrepancy between the input information and the GA value trend. In some embodiments, when it is estimated or determined that there is a discrepancy between the input information and the GA value trend, the system may avoid directly referring to the discrepancy.

In some embodiments, when there is little input, when there is a discrepancy in input information, or when it is determined that it is better to avoid direct reference, a message that does not directly refer to the matter may be generated and output to a subject. A message using an indirect expression about the matter or a message unrelated to the matter may be sent. Depending on the subject, directly referring to discrepancies in input information may reduce the motivation of the subject. In particular, when the personality of the subject cannot be clearly determined, communication with the subject may be performed in a mild mode. A style of communication may be optimized based on the personality of the subject, the changes of the subject over time, statistical data of a large number of subjects, and the like. The style of communication may be optimized using customer retention rate and/or a required rate of human counseling as evaluation indices. Communication styles will be further described later.

<Evaluation by Subject>

In some embodiments, the system may be configured to allow a subject to press a "like" or "dislike" button for videos, messages, GA value trends, and the like. In some embodiments, the system may be configured to allow a subject to input an evaluation in text or voice.

<Risk Determination>

The term "risk" as used herein generally refers to a possibility that an adverse event occurs and/or a possibility that a subject stops or suspends use of the service if the subject continues to use an unmanned service that only involves system use. The "possibility of an adverse event" includes that an adverse event is actually occurring, that an adverse event may occur in the future, and that an adverse event may have occurred in the recent past. The "possibility of an adverse event" includes a disease risk.

Examples of "disease risk" include, without limitation, hypoglycemia, complications of diabetes mellitus, infectious diseases, trauma, neoplastic lesions, and mental disorders such as depression, or the like.

<Analysis of Input Information>

In some embodiments, input information may be analyzed. Analysis results may be referred to for generation of various algorithms and output information and/or may be used for optimization thereof. The analysis results may be used for determination of risk. The analysis results may be used for determination of possibility of an adverse event, determination of disease risk, determination of customer retention rate, determination of dropout risk, and the like. Some examples are described below, but the present invention should not be interpreted as being limited thereto. An invention including those examples is possible, and an invention including examples other than those is also possible.

Analysis example 1) Determination of risk of hypoglycemia based on change in GA value

The subject is determined to have a risk of hypoglycemia in the following cases:

a GA value in a previous week is 20% or more and a GA value in a next week has decreased by 1.0% or more from the GA value in the previous week;
a GA value in a previous week is greater than 16% and less than 20% and a GA value in a next week has decreased by 0.6% or more from the GA value in the previous week; or
a GA value in a previous week is 16% or less and a GA value in a next week has decreased by 0.3% or more from the GA value in the previous week.

Analysis example 2) Determination based on answers to multiple-choice questions in "diary"

In questions with three choices "well done", "average", and "failed", when the rate of "failed" in the past week does not satisfy a predetermined value (for example, 30%), it is determined that there is a possibility of a need to change the goal, missed medications, or the like.

Analysis example 3) Determination based on free text input in "diary" or "message"

3-1) Input regarding physical condition

A comment indicating that there are subjective symptoms of hypoglycemia;
comments such as cold sweat, numbness/trembling of fingers, and strong hunger In these cases, it is determined that there is a possibility of hypoglycemia.
"A sudden increase/decrease in body weight"
"Inability to sleep", "No motivation", "Wanting to die", "Inability to stop crying" When such input is made, the system determines that there is a possibility of a mental disorder such as depression, and the like. When such input is made, it may be determined that urgency is high.

3-2) Input regarding mental burden

"Feeling easily tired", "Feeling unwell"
"Feeling stressed/pressured", "Being worried about not losing body weight"
"Feeling disappointed about bad results"
"Having to endure", "Having no pleasure"
"Having difficulty thinking of goals", "Remaining numerically unchanged despite efforts"

When such input is made, the system determines that there is a possibility of a mental disorder such as depression, and the like.

3-3) Input regarding diet therapy

"Reduced meal amount (extremely)", "Reduced carbohydrates (extremely)",
"Skipping meals (such as not eating staple food at dinner)", "Increased alcohol intake", "Restricting fluids"
When such input is made, the system determines that there is a possibility of hypoglycemia.

3-4) Input regarding pain

"Continuing pain after blood sampling",
"Having myalgia (such as lower back pain or knee pain) due to overexercise" When such input is made, the system determines that there is a possibility of an inflammatory disease (an infection or a tumor) or trauma.

3-5) Input regarding pharmacotherapy

"Increased/high frequency of missed medication doses"

When such input is made, the system checks for discrepancies with diary records, GA values, and the like. The system outputs a message prompting the subject to take medication or communicates with the subject via an indirect or unrelated message, as needed.

3-6) Spontaneous questions

"What should I do about XX?"
"Is it okay to continue my current behavior?"
"It seems ineffective. What do you think?"
"To what level should I decrease the GA value?"

When such questions are asked, the system may provide an appropriate educational video and provide appropriate advice via a message.

<Customer Retention Rate>

The system may obtain a rate at which the subject remains as an active user of the system, called a customer retention rate (CRR). The customer retention rate may be calculated over a certain period of time, such as one year, or the like. The customer retention rate is generally calculated by the following formula:

Customer retention rate (%) = ([Number of system users at end of period] - [Number of system users acquired during period])/[Number of system users at start of period] $\times$ 100

For example, the subject may voluntarily stop using the system. A subject directed to human counseling may stop using the system permanently or temporarily. For example, a subject directed to human counseling may be removed from a count of active users of the system. For example, a subject directed to human counseling may be formally counted as a user of the system.

In some embodiments, the system may change, adjust, or optimize output information so as to keep the customer retention rate at or above a threshold. Accordingly, for example, the motivation of the subject can be maintained. For example, the system may select educational information to be provided and/or generate a message to be provided so as to keep the customer retention rate at or above the threshold. For example, the system may optimize an algorithm for generation of educational information and/or an algorithm for generation of a message so as to keep the customer retention rate at or above the threshold.

In some embodiments, the system may provide human counseling to a subject as a premium service. That is, human counseling may be provided as a higher-level service compared to a service that only involves system use. In some embodiments, the customer retention rate may be determined based on subjects of the system in unmanned use excluding such premium-service users (subjects). In some embodiments, subjects of a human counseling service (premium service) may be included as retained subjects.

In some embodiments, the system may control the customer retention rate. Each algorithm may be optimized such that the customer retention rate can be a constant value.

Over a predetermined period of time, active users (subjects) who do not use human counseling or the like may be provided with mental, economic, or social benefits or incentives such as perks and bonuses.

In some embodiments, generation of beneficial information, generation or selection of educational videos, generation of messages, and generation of other output information may include use of artificial intelligence. In some embodiments, optimization of algorithms may include use of artificial intelligence. Algorithms such as for generation of output information and optimization of algorithms, and the like may be generated using artificial intelligence and may be further optimized using artificial intelligence each time new input information is obtained.

Artificial intelligence may be generative artificial intelligence. Artificial intelligence may use machine learning. Machine learning may use deep learning.

In some embodiments, a goal behavior proposed to the subject (proposed goal behavior) and information to be output to the subject may be generated in a communication style determined to be suitable for the subject.

The term "communication style" as used herein generally refers to a characteristic manner in which an individual communicates with others. As used herein, this term refers to a manner in which software or a system of the present application communicates with a subject, unless otherwise specified.

In some embodiments, a set including a plurality of communication styles may be prepared. In some embodiments, a plurality of sets may be prepared. In some embodiments, when a subject continues to use the system, the system may change from one communication style to another communication style in a set, or may change the set itself. In some embodiments, the system may add a new style to a set. A communication style may be based on various theories known at the time of filing of the present application, may be based on theories developed after the time of filing of the present application, may be based on independently developed theories, or may be based on a combination of a plurality of theories.

A communication style may be selected based on information related to a subject or the state of the subject. Examples of information related to a subject or the state of the subject include information input to the system by the subject spontaneously or as answers to questions, and the like measured values such as a GA value, and information, and the like lawfully obtained from a third party. More specific examples of information related to a subject or the state of the subject include, without limitation, a psychological state in a narrow sense of the subject (such as personality or a stage of behavioral change), knowledge of the subject, an environment of the subject, a usage history and a usage mode of software by the subject, a GA value of the subject, a GA value trend, and the like.

In some embodiments, a communication style may be selected, adjusted, or selected (hereinafter simply referred to as "selected") in accordance with a psychological state of a subject. A communication style may be selected so as to fit the glycemic management of the subject. For example, motivation for the glycemic management of the subject can be maintained, or a decrease in motivation can be avoided or suppressed. The subject can continue glycemic management using the software described in the present application over a long period of time without dropping out. A communication style may be selected in consideration of, in addition to the psychological state, a diabetic state and a lifestyle-related disease state.

In some embodiments, information to be output to a subject and the form of the output of the information, such as the content and style of linguistic information such as written language and spoken language; the content and style of information to be output to the subject such as (message) image information and video information such as moving images; the timing and period of time at which information is provided; the type of a combination of a plurality of information forms; and the like, are selected or optimized in accordance with a selected communication style.

In some embodiments, the content of information to be output or provided to a user or a subject (hereinafter simply referred to as "information"), or the timing and form of output and provision may be changed or adjusted in accordance with the communication style. In some embodiments, the content of a goal behavior proposed to a subject (proposed goal behavior) may be adjusted in accordance with the state of the subject. For example, as described below, in some cases it is effective to proactively output a certain amount of information, whereas in other cases it is effective to suppress the amount of information to be output. For example, for a subject who is able to take proactive actions, a proactive goal behavior may be proposed and a frequency of output to the subject may be set high. In contrast, in a case where taking proactive actions may lead to loss of motivation, a mild goal behavior may be proposed to the subject, and a frequency of output may be suppressed with attention to the responses of the subject.

Accordingly, a communication style may be selected in accordance with the communication style and in accordance with the state of the subject, and at the same time, the content of output information may also be adjusted. In some embodiments, the system may adjust a next goal setting to be proposed to a subject in accordance with the state of the subject. In this specification, even in the description of adjustment of a communication style, information to be output to a subject may also be adjusted, unless otherwise specified. In some examples, a communication style and the content of output information need not be regarded as independent concepts, and adjustment of the output information itself may be interpreted synonymously with adjustment of the communication style as an integral whole.

The state of the subject may be constant over a long period of time. However, in many cases, occurrence of some event or the like may trigger a discontinuous change in the state of the subject over a short period of time. Alternatively, the state of the subject may change even over several months or several years. Accordingly, the system may always or frequently grasp the state of the subject, which can change dynamically. Based on the state of the subject at each point in time and a GA value or a GA value trend, the system may dynamically adjust, select, determine, or optimize a communication style (in this specification, the term "determine" may be used simply).

In some embodiments, the system may infer a psychological state of a subject.

In some embodiments, a method may include a step of inferring a psychological state of a subject. A communication style may be classified according to the inferred psychological state of the subject. That is, a communication style may be selected based on the psychological state of the subject.

The term "psychological state" as used herein generally refers to a state or classification known in psychology, communication theory, or fields related to psychology. A psychological state of a subject may be classified based on various theories. Examples of theories used for a communication style include, without limitation, personality typology and personality trait theory, or the like. For example, the Big Five theory may be used as a trait theory. These are merely illustrative, and use of other theories should not be excluded. Not only various psychological theories or hypotheses known at the time of filing of the present application but also various psychological theories or hypotheses that have become known since then may be used.

For example, examples of a psychological state of a subject include, without limitation, personality of the subject, knowledge related to glycemic management of the subject, and a stage of behavioral change of the subject. Some or all of "knowledge" and other information related to a subject may be treated as a psychological state in some embodiments, and may be treated as a non-psychological state in other embodiments.

As used herein, the Japanese term "jinkaku" should not be interpreted in a limiting manner and may include meanings of "seikaku" and "kosei" in Japanese or be used interchangeably therewith, and generally means "personality" in English as a psychological term. Examples of personality include, without limitation, cooperativeness and a tendency to follow authority figures, or the like.

"Personality" may be evaluated by conducting a test. A personality test may be conducted by a human, and the result may be input to the system by an implementer of the test or by a subject who is the target of the test. The system may perform a personality test on the subject and obtain a result by input to the system by the subject or by sensing some reaction of the subject.

For example, "personality" may be classified or expressed by traits based on trait theory. For example, classification may be made by the Big Five personality traits. "Personality" may be classified based on theories used in the fields of psychology and behavioral science, for example, psychoanalysis, behaviorism, social cognitive theory, and humanistic psychology, and the like.

For example, in social cognitive theory (SCT), information regarding a subject may be obtained regarding the elements "personal", "behavior", and "environment", which are considered to mutually influence one another in a triadic causation model.

For example, regarding "personal", the personality of the subject may be tested. For example, whether the cooperativeness of the subject is high or low; and an attitude toward authority, namely, whether the subject follows instructions of persons generally regarded as authorities such as physicians and healthcare professionals, or the like, and whether the subject tends to acquire and implement independent knowledge may be tested.

For example, regarding "behavior", information regarding tendencies or experiences of the subject may be acquired. For example, it may be tested whether the subject can or does cook, whether the subject often forgets to take medications, whether the subject actively participates in treatment, and whether the subject has successful experiences, and the like.

For example, regarding "environment", it may be tested whether the subject is in an environment in which behavioral change is easy or difficult to occur. For example, it may be tested whether the subject has events that can hinder behavioral change (presence or intensity of stress, life rhythm, presence or absence of disease, and the like) and whether a spouse or a housemate cooks, and the like.

A state of a subject and/or a communication style may be determined based on techniques of communication theory, for example, without limitation, a social style theory. Based on various input information or acquired information, the subject may be classified into any social style of amiable, analytical, expressive, or driving in the social style theory. In some embodiments, a communication style may be selected in accordance with the social style of the subject.

The system may classify a subject in any stage of behavioral change in a transtheoretical model (TTM). Generally, subjects are classified into any of five stages of behavioral change (precontemplation, contemplation, preparation, action, and maintenance). In some embodiments, a communication style may be selected in accordance with the stage of behavioral change of the subject.

The system may acquire knowledge of the subject, namely, information that the subject has regarding glycemic management. In some embodiments, the system may administer a test regarding glycemic management to the subject and acquire results thereof. For example, character information, educational information such as videos, and other information provided by the system to the subject may be regarded as knowledge acquired by the subject or as the literacy of the subject. For example, the system may test whether the subject understands information provided to the subject. The system may further evaluate the knowledge or literacy of the subject, based on results of the test. For example, when the subject watches a high-difficulty video and presses "Like", it may be determined that the amount of knowledge of the subject is high. In some embodiments, a subject inputs to the system information acquired without using the system, and the system may accordingly acquire the knowledge of the subject. The system can more accurately grasp the knowledge of the subject and can reinforce knowledge information suited to the subject. In some embodiments, a communication style may be selected in accordance with the knowledge or literacy of the subject.

The system may adjust, select, determine, or optimize a communication style, based on a usage mode of the system, including input to the system by the subject, or a usage history of the system. In some embodiments, the system may analyze the frequency of use of the system by a subject. When the frequency of use of the system is high, it may be inferred that the subject is proactive and likely to undergo behavioral change, or the subject is likely to be able to maintain appropriate glycemic management. In some embodiments, the mode in which the subject responds to output information from the system to the subject may be analyzed. For example, the time taken from notification of a GA value to the subject until the subject confirms the GA value may be analyzed. For example, the time taken from notification of provision of a video, a message, or the like until the subject confirms the content thereof may be analyzed. For example, the frequency at which, in response to notification of provision of a video, a message, or the like, the subject confirms the content thereof may be analyzed. For example, the frequency at which the subject spontaneously accesses the system may be analyzed. In some embodiments, the system may analyze the amount of input to the system by the subject. For example, the amount of character information (for example, number of characters) input into a diary or the like by the subject may be analyzed. For example, it may be inferred that the shorter the times described above are, the higher the frequency is, or the larger the amount is, the higher the motivation of the subject is, and that the longer the times described above are or the lower the frequency is, the lower the motivation of the subject is. Alternatively, the motivation of the subject may be inferred from a combination of the values described above.

In fact, according to clinical studies conducted by the applicant, there was a significant difference in the number of characters (in Japanese) input into a diary ($p = 0.027$) between a group in which a GA decrease was greater than the average ($\Delta GA < -1.35\%$) and a group in which a GA decrease was the average or less ($\Delta GA \geq -1.35\%$). This result suggests a possibility that a greater average number of characters in a diary correlates with improvement in GA value due to behavioral change (Fig. G1). It is considered that input to the system helps improve the motivation of the subject.

As described above, based on the amount of input to the system, a level of motivation, literacy, a stage of behavioral change or a possibility of change thereof, a possibility of improvement or maintenance of a GA value, and the like may be inferred for the subject. Fig. G1 illustrates merely one example. From amounts, frequencies, and other forms of various inputs to the system by the subject, a change in GA value and further a state of the subject (psychological state, literacy, stage of behavioral change) can be inferred.

In some embodiments, the system may adjust, select, determine, or optimize a communication style, based on the degree of achievement of a goal set voluntarily by the subject. For example, it may be inferred that the higher the degree of achievement of a set goal is, or the larger the amount and frequency of self-evaluation input to the system are, the higher the motivation of the subject is.

In some embodiments, the system may adjust, select, determine, or optimize a communication style in accordance with a GA value trend (such as whether a GA value is decreasing or whether a GA value is maintained within an appropriate range). For example, when a GA value is trending downward or is maintained within an appropriate range, it may be inferred that the motivation of the subject is high. For example, when the degree of achievement of a set goal is high and a GA value is trending downward, it may be inferred that the motivation of the subject is high. On the other hand, when the degree of achievement of a set goal is low although a GA value is trending downward, the behavior of the subject may be highly evaluated, but the literacy of the subject may be inferred to be low.

The system can acquire information related to the subject, which is necessary for determining a communication style, in various forms. For example, the system can directly acquire information input to the system by operation performed by the subject or a user other than the subject. For example, a medical interview may be conducted for the subject by a human or automatically by the system, and the system may acquire results thereof. For example, the subject's operations (input and output) may be analyzed to obtain information related to the subject. The amount of input, such as the number of characters in a diary, time information of input, time of day, frequency, monotony or variety of input information, and the like may be analyzed. For example, daily self-evaluation, which varies from day to day rather than being monotonous, may be associated with behavioral change of the subject and a decrease or maintenance of GA.

The system may acquire image information and voice information of the subject. The image information includes, for example, still images or moving images of the subject's face, upper body, or entire body by a camera, and facial expressions, movements, and the like can be analyzed therefrom. The image information can be acquired from an electronic terminal, or the like used by the subject. The voice information includes, for example, voice input of the subject to the system and conversations with others, or the like and a speech rate, a voiceprint, and the like can be analyzed therefrom. The system may acquire biometric information or activity information from a wearable device of the subject. Biological information such as heart rate, or the like exercise information, diet information, and the like can be acquired and analyzed.

For example, when starting to use the system, the subject may undergo a medical interview or a test. In some embodiments, the medical interview may be conducted at a plurality of timings. Examples of the timings include, without limitation, the start of the system, when a GA value is provided, when the subject uses the system, and when the subject inputs something to the system, or the like.

Conducting a large number of medical interview questions at once may reduce the motivation of the subject. In some embodiments, the system may administer a plurality of types of medical interviews to the subject over a plurality of sessions. Usually, one medical interview is made up of a plurality of questions (a set of questions). In some embodiments, the system may execute a plurality of questions, which constitute one medical interview, at a plurality of timings in small parts. For example, for each of the plurality of questions constituting a medical interview, a degree of efficacy or immediacy may be defined or given as an attribute. For example, a question having high efficacy may be assigned a high priority. Questions may be asked to the subject in order of priority. That is, the system may pose high-priority questions first. In some embodiments, the priority may be fixed. In some embodiments, the priority may be variable, and the system may dynamically change the priority of each question, based on other input information. Therefore, it is possible to, for example, maintain the motivation of the subject and more effectively assist the glycemic management of the subject.

Generally, management of diet is most effective for glycemic management. Accordingly, questions regarding diet may be assigned a high priority. For example, questions regarding diet may be assigned a higher priority than questions regarding matters other than diet, specifically, questions regarding exercise or questions regarding medication. That is, medical interview questions regarding diet may be asked, or a goal regarding diet may be proposed. While the subject is allowed to feel the effect thereof, a next-priority question (for example, a question regarding exercise) may be asked.

<Examples>

The following describes an example in which a communication style for conveying a goal behavior indicating "avoiding sweet soft drinks" to a subject, and output information are changed in accordance with a state (types 1 to 3) of the subject. As summarized in Table 1, types 1 to 3 having respective characteristics were assumed as states of subjects.

[Table 1]

| Characteristics | Type 1 | Type 2 | Type 3 |
|---|---|---|---|
| Willingness To Improve | Proactive in treatment | Present | Present |
| Cooperativeness | High | Not high | High |
| Literacy | High | High | Not high |
| Tendency to follow authority figures | Present | Tendency to resist authority figures | Present |
| Preferred communication style | N.A. | N.A. | Simple |
| Stage of behavioral change | From preparation to action | Action | Action |

Type 1: The subject is proactive in treatment, has high cooperativeness and literacy, tends to follow instructions of authorities (has a willingness to listen to and act on what healthcare professionals and experts say), and a stage of behavioral change is estimated to be from preparation to action. Accordingly, a specific and easy-to-implement proposal and reasons therefor may be generated as output information. For example, the following proposal may be output to the subject.

"When you are thirsty, it is good to choose water or unsweetened tea. It is known that sugary drinks tend to raise blood glucose levels. There is also a theory that even sugar-free soft drinks with artificial sweeteners are not good for glycemic management. Would you try to see if glycemic management improves with water or tea?"

Type 2: The subject has a desire to improve and high literacy, but has low cooperativeness, tends to resist authority figures, and is trying independent therapies, and a stage of behavioral change is estimated to be action. Accordingly, a proposal indicating an attitude of empathizing with the feelings of the subject and voluntary judgment may be generated. For example, the following proposal may be output to the subject.

"Water and unsweetened tea may be the optimal drinks for your current glycemic management. Although not widely known, even sugar-free soft drinks with artificial sweeteners, such as sugar-free cola, are said sometimes to be not good for glycemic management. Would you try it and see if you can find the glycemic management method that is most effective for you?"

Type 3: The subject has a desire to improve, has high cooperativeness, tends to follow authorities, but does not necessarily have a large amount of knowledge, and is estimated to prefer simple communication, and a stage of behavioral change is estimated to be action. Accordingly, a concise and specific proposal may be generated. For example, the following proposal may be output to the subject.

"For drinks, water or unsweetened tea is recommended! Please avoid sweet drinks."

In some embodiments, an output to a subject may be adjusted based on information related to the subject. For example, a correlation of a plurality of information items related to the subject may be recognized, and output information may be generated based on the correlation. For example, output information may be generated based on consistency or discrepancies (mismatches) among a plurality of information items related to the subject.

When a GA value is a value to be improved, behavioral change is required. However, directly requesting the subject to change behavior may cause psychological resistance and is not effective in many cases. Behavioral change, particularly behavioral change related to lifestyle, is considered to be influenced by a psychological state in a broad sense. Accordingly, a correlation between a behavior of the subject that can affect a GA value and a state of the subject (in particular, a psychological state including knowledge) may be recognized. Working on a psychological state related to a behavior of the subject in an effective communication style can induce a behavioral change of the subject, for example, naturally and without a sense of resistance.

<Examples>

An example 10000 of a process of adjustment of a communication style suitable for a subject and communication with the subject based on the communication style, which is performed by the system, will be described with reference to Fig. G2.

The system acquires information regarding a subject (10100). These pieces of information are information input to the system or information acquired by the system from outside, and are collectively referred to as input information. Examples of information that the subject inputs to the system by themselves include, without limitation, a goal (weekly goal) set by the subject themselves, a diary, a self-evaluation, or a reaction to information provided by the system (for example, pressing a "like" button). Examples of information received by the system or acquired from outside include, without limitation, a GA value of the subject, answers to medical interview questions, healthcare data of the subject, which is obtained by a wearable device, or the like and health-related data of the subject, which is lawfully obtained from a third party (for example, a medical institution or a health checkup period).

Behavior of glycemic parameters including a GA value, behavioral information including diet, exercise, and medication, and correlations therebetween are stored in a database 10210 so as to be accessible from the system. The system accesses the database 10210 and lists (10310), from the data, behavioral changes or goal behaviors that match input information of the subject and are effective for the glycemic management of the subject. The behaviors ("behavior 1", "behavior 2", ...) in the list of goal behaviors may be assigned priorities.

On the other hand, psychological models that can be used by the system and psychological data related thereto are stored in a database 10220 so as to be accessible from the system. The system accesses the database 10220 and refers to input information of the subject to infer (10320) a state of the subject (such as a psychological state of the subject, personality, an environment, a behavior pattern, knowledge or literacy related to glycemic management, or a stage of behavioral change of the subject).

The system may recognize a correlation between the state of the subject (10320) and a current behavior (10100) of the subject or a goal behavior (10310) to be taken by the subject.

The system determines a communication style (10400) to be taken for the subject, based on the state of the subject (10320) and, if necessary, an appropriate psychological model (10220).

The system generates output information (10500) in accordance with the determined communication style (10400). More specifically, the system generates a goal behavior to be taken by the subject (proposed goal, 10510) and, other than the proposed goal, for example, a message to the subject, video information, voice information, and other output information (10520). For example, for generation including generation of encouragement, it is effective to sufficiently reflect the communication style. Furthermore, an output mode of the output information is also determined. The proposed goal is displayed to the subject at regular timings such as display of a latest GA value. However, provision of the other information to the subject can be irregular. It is therefore effective to appropriately adjust and determine the amount of information per output, the timings of output, and the like in accordance with the communication style.

<Examples>

The following describes specific examples created by processing actual cases. The system recognized that a GA value of a subject should be improved. The system acquired, as information related to behavior of the subject, information that "the subject eats three mandarin oranges as a snack once a day". Further, the system acquired, as information regarding knowledge of the subject, information that "the subject recognizes that mandarin oranges are low in sugar". The system may acquire such information from input to the system by the subject or a user, acquire such information in a medical interview for the subject, or lawfully acquire such information from a third party.

The system recognized that sugar intake was included in a list of behaviors effective for improvement of GA. The system also recognized that mandarin oranges contain a high amount of sugar. From this, the system inferred that, in relation to sugar, intake of mandarin oranges might be increasing the blood glucose levels of the subject. Accordingly, the system inferred that, if a behavioral change of restricting intake of mandarin oranges were realized by the subject, the possibility of decreasing the GA value would be high.

On the other hand, the system determined that the current intake of mandarin oranges was correlated to the knowledge of the subject that "mandarin oranges are low in sugar". More specifically, it was determined that a behavior that "the subject eats three mandarin oranges as a snack once a day" was supported by the recognition that "mandarin oranges are low in sugar".

The system can adjust output information to be presented as a proposal to the subject in order to induce a behavioral change of refraining from intake of mandarin oranges as a snack, based on a psychological state of the subject. Accordingly, the system determined a communication style for each type of subject and generated a goal behavior to be specifically proposed, using the communication style. As one example, the system generated output information as follows for each classification of subjects based on a social style theory.

- When the subject is of an amiable type, a specific and easy-to-implement proposal and a reason therefor are conveyed with care in a manner that empathizes with feelings, such as "Eating even mandarin oranges in large amounts may lead to an increase in blood glucose level. Would you reduce mandarin oranges to about one per day and replace the rest with nuts or cheese?"
- When the subject is of an expressive type, a specific goal behavior is proposed, such as "Please limit mandarin oranges to one per day, and then try nuts or cheese as snacks".
- When the subject is of a driving type, new knowledge and a proposal of a change of goal behavior are conveyed, such as "It is known that eating many mandarin oranges tends to raise blood glucose levels. If approximately one mandarin orange is eaten per day, blood glucose levels will not rise so much. Nuts and cheese have less sugar".
- When the subject is of an analytical type, objective information is provided and the decision is left to the subject, such as "It is said that if approximately one mandarin orange is eaten per day, blood glucose levels will not rise so much. Nuts and cheese, which have less sugar, are also recommended according to your preference".

As in the above examples, in some embodiments, the system may recognize a correlation between a behavior of a subject and a GA value of the subject, recognize a correlation between the behavior of the subject and the related state of the subject (such as a psychological state, knowledge, or a stage of behavioral change), determine a communication style according to the type of the subject, and generate output information such as a proposed goal behavior and appropriate knowledge. The system may always collect the content of behaviors, input information, external information, and the like and infer a state of the subject or infer a change in the state of the subject when a change is recognized in the collected information. It is possible to assist glycemic management of the subject by grasping a state of the subject and adapting a communication style.

<Optimization of Communication Style>

Effects of output information provided from the system to the subject and a communication style selected at that time can be evaluated by various methods. Then, the evaluation can be used to optimize a decision algorithm of the system.

When it is evaluated that a communication style or output information was effective, a similar algorithm may continue to be used, and the information may be used for machine learning of the algorithm. Examples in which it may be evaluated that a communication style was effective include, without limitation:

- The subject themselves set a goal that is the same as or similar to a proposed goal behavior.
- After a proposal of a goal behavior, reactions (inputs, such as inputs of "like" and diary inputs) to input to the system by the subject or to signals from the system increased or improved.
- A GA value to be improved was improved, or a GA value was maintained within an allowable range.

When it is evaluated that a communication style or output information was not effective, an algorithm may be changed or optimized. Examples in which it may be evaluated that a communication style was not effective include, without limitation:

- The subject themselves set a goal completely different from a proposed goal behavior or a goal based on a misunderstanding.
- After a proposal of a goal behavior, reactions (inputs, such as inputs of "like" and diary inputs) to input to the system by the subject or to signals from the system decreased or deteriorated.
- A GA value to be improved did not improve or deteriorated.

In some embodiments, when it is evaluated that a communication style was not effective, the system may change or modify some part of the algorithm. For example, it may be inferred that a psychological test such as a personality assessment, and the like was not properly performed, and the test may be performed again or another test may be performed.

In some embodiments, when it is evaluated that a communication style was not effective, the system may notify the subject or a user that human counseling should be received. Some part of the algorithm may be changed or modified. In some embodiments, human counseling may be proposed to the subject or the like when an evaluation of no effect of a communication style has been made only once. However, in some embodiments, in some embodiments, human counseling may be proposed to the subject or the like when an evaluation of no effect of a communication style has been made a predetermined number of times. Rather than making a drastic policy change, watching over the subject for a relatively long span of time is sometimes important.

<Description of Process Sequences>

Hereinafter, process sequences 1000 to 8000 of the system according to some embodiments will be described with reference to Figs. A1 to A8.

<Embodiment 1>

Fig. A1 illustrates a process sequence 1000 of the system according to an embodiment.
The subject may proactively set an initial goal. The subject inputs the initial goal to the system. The system acquires the input initial goal (1111).
The subject measures a GA value. A GA value measuring system (not illustrated) sends GA value information to the system. The system receives and acquires the GA value (1210). The system also refers to a previous GA value to generate a GA value trend and outputs the GA value trend to the subject (1220). The subject receives the GA value trend and can recognize a change in their own GA value.
The system generates a next-week goal and outputs and proposes the next-week goal to the subject (1120). When the system determines that the goal is not changed, the unchanged goal is sent to the subject. The subject re-recognizes their own goal and a fresh awareness is maintained.
To change the goal, the subject resets the goal and inputs a new goal to the system. The system acquires the input new goal (1112).
When next proposing a goal (1120), the system may refer to the newly acquired goal (1112). Thereby, a new next-week goal may be generated.
When a new GA value is acquired (1210), the system generates a GA value trend (1220) and sends the GA value trend to the subject. At this time, the system may simultaneously send a new next-week goal to the subject.

<Embodiment 2>

Fig. A2 illustrates a process sequence 2000 of the system according to an embodiment. In the process sequence 2000, a GA value trend (2220) generated from an acquired GA value (2210) is referred to in order to generate a next-week goal (2120).
To change the goal, the subject resets the goal and inputs a new goal to the system. The system acquires the input new goal (2112).
When next proposing a goal (2120), the system may refer to the newly acquired goal (2112) and the GA value trend (2220). Thereby, a new next-week goal may be generated.

<Embodiment 3>

Fig. A3 illustrates a process sequence 3000 of the system according to an embodiment. The system outputs beneficial information to the subject (3321). In the process sequence 3000, the beneficial information includes educational information (3321). For example, the educational information may be provided in the form of a video clip. For example, the system may hold a large number of video clips in advance or have a large number of video clips in an accessible way and may select, from among them, a video clip to be provided.
The educational information may be provided to the subject in accordance with some input to or output from the system. For example, the educational information may be provided together with output of a goal, a GA value trend, and the like. For example, when the subject starts or opens an application related to the system, the system may sense that the terminal has been operated, triggering the provision of educational information. The video clip may be blocked or ignored when it is determined that the subject will not watch the video clip.
For selection of educational information (3321), an initial goal (3111) and a goal set thereafter or set newly (3112) may be referred to. For selection of educational information (3321), a GA value trend (3220) generated from an acquired GA value (3210) may be referred to. For a proposal of a next-week goal (3120), the initial goal (3111), the goal set thereafter or set newly (3112), and/or the GA value trend (3220) may be referred to.

<Embodiment 4>

Fig. A4 illustrates a process sequence 4000 of the system according to an embodiment. The system outputs beneficial information to the subject (4322). In the process sequence 4000, the beneficial information includes a message (4322). For example, the message may be provided in the form of text. The message may be configured such that a goal is achieved and/or a GA value trend improves. The message can provide motivation for the subject to maintain behaviors to improve lifestyle.

For generation of the message (4322), an initial goal (4111), a goal set thereafter or set newly (4112), and/or a GA value trend (4220) generated from an acquired GA value (4210) may be referred to. For a proposal of a next-week goal (4120), the initial goal (4111), the goal set thereafter or set newly (4112), and/or the GA value trend (4220) may be referred to.

<Embodiment 5>

Fig. A5 illustrates a process sequence 5000 of the system according to an embodiment. The system acquires behavior-related information of the subject (5410). For generation of a message (5322), an initial goal (5111), a goal set thereafter or set newly (5112), a GA value trend (5220) generated from an acquired GA value (5210), and/or the behavior-related information (5410) may be referred to. For a proposal of a next-week goal (5120), the initial goal (5111), the goal set thereafter or set newly (5112), the GA value trend (5220), and/or the behavior-related information (5410) may be referred to.

Accordingly, generation of a message can be optimized. For the subject, the message is one of important windows that can form a sense of familiarity with the system. Sending an appropriate message according to the status of the subject can, for example, encourage the subject to continue using the system and thereby improve the glycemic control of the subject.

<Embodiment 6>

Fig. A6 illustrates a process sequence 6000 of the system according to an embodiment. The system outputs various kinds of information, namely, a next-week goal (6120), educational information (6321), a GA value trend (6220) generated from an acquired GA value (6210), a message (6322), and the like, to the subject. In the process sequence 6000, the subject can react to these. For example, the subject can input an evaluation regarding these pieces of information output from the system. The system acquires the input evaluation from the subject. In the process sequence 6000, the system can acquire an evaluation (6230) regarding a GA value trend, an evaluation (6331) regarding the educational information (6321), and/or an evaluation (6332) regarding a message.

From these evaluations (6230, 6331, and 6332), a status regarding behavioral change of the subject (including, for example, feelings, ways of thinking, reactions, behaviors, and the like) can be acquired. The system analyzes these evaluations (6520).

Additionally or alternatively, in the process sequence 6000, the system analyzes (6520) other inputs, namely, an initial goal (6111), a new goal (6112), a GA value (6210), and/or behavioral information (6410).

The information input from the subject is useful for generation of information to be output next, or the like. For a proposal of the next-week goal (6120), selection of the educational information (6321), and/or generation of the message (6322), the system may refer to analysis results of the input information (6520).

<Embodiment 7>

Fig. A7 illustrates a process sequence 7000 of the system according to an embodiment. The system optimizes algorithms of various processes (7620).

In the process sequence 7000, the system performs a plurality of processes, namely, a proposal of a next-week goal (7120), selection of educational information (7321), generation of a GA value trend (7220) generated from an acquired GA value (7210), generation of a message (7322), and/or analysis of input information (7520). Outputs from the system to the subject, namely, an initial goal (7111), a proposal of the next-week goal (7120), provision of the educational information (7321), and provision of the message (7322), and inputs from the subject to the system, namely, a new goal (7112), behavior-related information (7410), and evaluations (7331, 7230, and 7332) regarding the outputs, are useful information regarding behavioral change of the subject. Based on these pieces of information, algorithms for generation of information to be output, namely, a proposal of a next-week goal (7120), selection of educational information (7321), generation of a GA value trend (7220), and generation of a message (7322), can be dynamically changed or optimized (7620).

Accordingly, for example, the motivation of the subject can be maintained or improved, a GA value of the subject can be improved in the short term or the long term, or deterioration can be avoided or suppressed. For example, a probability that the subject will use the system over a long period of time can be increased.

<Embodiment 8>

Fig. A8 illustrates a process sequence 8000 of the system according to an embodiment. The system may have various tuning functions.

In some embodiments, a risk of the subject may be determined (8720). For example, a risk of the subject may be determined (8720) with reference to a GA value trend (8220) generated from an acquired GA value (8210) and/or analysis results of input information (8520). For example, when the GA value trend medically indicates symptoms or signs of a disease or the like, it may be determined that there is a high probability that the subject has a risk. Based on the determination result (8720), it may be determined whether the subject should be made to receive human counseling (or an interview) or whether the subject should be allowed to continue using the system involving no human intervention (8721). When it is determined (8721) that the subject should receive an interview, the system may notify the subject of information indicating that the subject should receive an interview.

In some embodiments, a customer retention rate (CRR) may be obtained (8820).

The subject can transition to human counseling based on the risk determination (8720). For example, the subject can voluntarily stop using the system. A rate at which the subject continues as a user of the system, called a customer retention rate, thus indicates that there exist subjects who leave the system. It is important to keep the customer retention rate at or above a certain threshold.

In some embodiments, the motivation of the subject may be maintained so as to keep the customer retention rate at or above a threshold. For example, educational information to be provided may be selected (8321) and/or a message to be provided may be generated so as to keep the customer retention rate at or above a threshold. For example, an algorithm for generation of educational information (8321) and/or an algorithm for generation of a message (8322) may be optimized (8620) so as to keep the customer retention rate at or above a threshold or by using the customer retention rate as an evaluation index.

In some embodiments, the customer retention rate may be controlled. Each algorithm may be optimized such that the customer retention rate can be a constant value (8620).

<Example 1: Example of GA Value Trend>

Fig. B1 illustrates an example of display formats of GA value trends. In Fig. B1, GA values at weeks "1", "2", and "3" on a horizontal axis are schematically illustrated. A unit on a vertical axis is any unit and schematically represented, and does not correspond to real values. In graphs, two consecutive changes in GA value are classified into three types, namely, increase (up, "U"), decrease (down, "D"), and no change (flat, "F"). Behavior of three GA values can be represented by classifying each of the two changes into these three types and is classified into nine types: {F, F}, {F, U}, {F, D}, {U, F}, {U, U}, {U, D}, {D, F}, {D, U}, and {D, D}.

Here, each point may be a consecutive weekly GA value. However, other definitions are also possible. For example, these points may be three GA values having intervals of a plurality of weeks. For example, each point may be defined by a statistical value (such as a mean value) of GA values acquired over a plurality of weeks, and three points may be displayed in a time series.

A GA value trend may be provided to a subject and, together with the GA value trend, an evaluation thereof may be provided as text information. In some embodiments, a short-term trend may be evaluated. For example, changes among GA values over three weeks (or three times), namely, two changes, may be evaluated. In some embodiments, a long-term trend may be evaluated. For example, a change between a GA value at the time of starting to use the system (initial GA value) and a latest GA value may be evaluated. These evaluations may be classified into predetermined types, may be described in natural language, or may be configured by a combination of them.

Table 2 illustrates an example of evaluation of a short-term trend. Two weekly changes of changes over three weeks (a change from week n-2 to week n-1 and a change from week n-1 to week n), a typological evaluation thereof ("positive", "neutral", or "negative"), and a sentence pattern for each, which is to be output to a subject, are illustrated. Weekly changes are classified into the nine types as illustrated in Fig. B1. Expressions of weeks used for evaluations, classifications of evaluations, and basic sentence patterns described in Table 2 are non-limiting examples. The present invention can also be interpreted as embodiments that are based on this table. However, the present invention should not be interpreted as being limited by the description of Table 2.

[Table 2]

| Examples of evaluations of short-term changes and messages Evaluations of weekly changes, and sentence patterns in messages | | | |
|---|---|---|---|
| Weekly change | | Evaluation | Basic sentence pattern 1 |
| (n-2)->(n-1) | (n-1)->n | | |
| Down | Down | Positive | It has decreased for two consecutive weeks! Excellent!! |
| Flat | Down | Positive | It has begun to decrease! Keep it up! |
| Up | Down | Positive | You managed to recover somehow! This is a good trend! |
| Down | Flat | Neutral | The decrease has leveled off. |
| Flat | Flat | Neutral | The current state is being maintained. |
| Up | Flat | Neutral | You managed to hold steady. |
| Down | Up | Negative | It has rebounded. |
| Flat | Up | Negative | It is showing an upward tendency. |
| Up | Up | Negative | It has increased for two consecutive weeks... |

Table 3 illustrates an example of evaluation of a long-term trend. A change between a GA value at the time of starting to use the system and a latest GA value is classified into 12 types according to an amount thereof. An evaluation ("improvement", "flat", or "deterioration") and a sentence pattern to be output to a subject for each classification are illustrated. The amounts of change, the number of classifications, classifications of evaluations, and expressions of basic sentence patterns described in Table 3 are non-limiting examples. The present invention can also be interpreted as embodiments that are based on this table, but the present invention should not be interpreted as being limited by the description of Table 3.

[Table 3]

| Examples of evaluations of long-term changes and messages Evaluations of changes from start, and sentence patterns messages | | |
|---|---|---|
| Change from start | Evaluation | Basic sentence pattern 2 |
| - 4.0% ~ | Improvement | Remarkably, it has decreased by 4 or more from the starting point. If this trend continues, it will result in an improvement of approximately 1.0 in HbA1c. |
| - 3.0 % ~ - 4.0 % | Improvement | It has still decreased by 3 or more from the starting point. Continuing this for 1 to 2 months will improve HbA1c by 0.7 to 0.8. |
| -2. 0% ~ - 3.0% | Improvement | It has decreased by 2 or more from the starting point. Continuing the 3% improvement in GA for 1 to 2 months will improve HbA1c by 0.7 to 0.8. |
| -1. 0% ~ - 2.0% | Improvement | It has decreased by 1 or more from the starting point. Continuing the 2% improvement in GA for 1 to 2 months will improve HbA1c by 0.5 to 0.6. |
| - 0.3 % ~ - 1.0% | Improvement | A slight improvement from the starting point is observed. Continuing the 1% improvement in GA for 1 to 2 months will improve HbA1c by 0.2 to 0.3. |
| - 0. 3 % ~ + 0. 1 % | Flat | It remains almost unchanged from the starting point. |
| + 0.1% ~ + 0.3% | Flat | A slight deterioration from the starting point appears to be observed. |
| + 0.3% ~ + 1.0% | Deterioration | A moderate deterioration from the starting point is observed. If the 1% deterioration in GA continues for 1 to 2 months, HbA1c will worsen by 0.2 to 0.3. |
| + 1.0% ~ + 2.0% | Deterioration | It has deteriorated by 1 or more from the starting point. If the 2% deterioration in GA continues for 1 to 2 months, HbA1c will worsen by 0.5 to 0.6. |
| + 2.0% ~ + 3.0% | Deterioration | It has deteriorated by 2 or more from the starting point. If the 3% deterioration in GA continues for 1 to 2 months, HbA1c will worsen by 0.7 to 0.8. |

(continued)

| Examples of evaluations of long-term changes and messages<br>Evaluations of changes from start, and sentence patterns messages | | |
|---|---|---|
| Change from start | Evaluation | Basic sentence pattern 2 |
| + 3.0% ~ +4. 0 % | Deterioration | It has deteriorated by 3 or more from the starting point. Continuing this for 1 to 2 months will worsen HbA1c by 0.7 to 0.8. |
| + 4.0 % ~ | Deterioration | It has deteriorated by 4 or more from the starting point, which is concerning. |

A relationship between a short-term trend and a long-term trend can be classified into conjunctive, adversative, or neither. To connect two sentences in a way natural for a subject, a conjunction to be inserted between the two sentences may be appropriately selected. Table 4 illustrates conjunctions that connect short-term trend messages (Table 2) and long-term trend messages (Table 3). Examples of conjunctions described in Table 4 and evaluations of the short-term trend and the long-term trend, and the like to be referred to in order to select a conjunction are non-limiting examples. The present invention can also be interpreted as embodiments that are based on this table, but should not be interpreted as being limited by the description of Table 4.

[Table 4]

| Examples of expressions for connecting short-term trend messages and long-term trend messages | | |
|---|---|---|
| Evaluation of short-term trend | Evaluation of long-term trend | Conjunction between basic sentence pattern 1 and basic sentence pattern 2 |
| Positive | Improvement | ( ^ - ^ ) |
| Positive | Flat | Nevertheless |
| Positive | Deterioration | Nevertheless |
| Neutral | Improvement | Moreover |
| Neutral | Flat | Also |
| Neutral | Deterioration | Nevertheless |
| Negative | Improvement | Nevertheless |
| Negative | Flat | Nevertheless |
| Negative | Deterioration | Moreover |

Based on the classifications and sentence patterns illustrated in Tables 2 and 3 and the conjunctions illustrated in Table 4, GA value trends can be classified into $9 \times 13 = 108$ types, and natural language expressions (messages from the system to the subject, system messages) corresponding to the respective types can be generated. Messages generated based on Tables 2 to 4 relatively objectively represent GA value trends.

In some embodiments, a message including future guidelines, guidance, psychological support, and the like may be generated with respect to a GA value trend. Table 5 illustrates an example of messages including guidance for the subject, based on nine combinations of short-term trends (positive, neutral, and negative) and long-term trends (improvement, flat, and deterioration). Examples of sentence patterns described in Table 5 and evaluations of the short-term trends and the long-term trends, and the like to be referred to in order to select classifications thereof are non-limiting examples. The present invention can also be interpreted as embodiments that are based on this table, but should not be interpreted as being limited by the description of Table 5.

[Table 5]

| Examples of guidance messages based on short-term trends and long-term trends | | |
|---|---|---|
| Evaluation of short-term trend | Evaluation of long-term trend | Basic sentence pattern 3 |
| Positive | Improvement | Keep it up! Let's record in your review what has been effective! |

(continued)

| Examples of guidance messages based on short-term trends and long-term trends | | |
|---|---|---|
| Evaluation of short-term trend | Evaluation of long-term trend | Basic sentence pattern 3 |
| Positive | Flat | Some adjustments may be needed to reach your target value. Please make use of consultations with your supporter. |
| Positive | Deterioration | For better management of your daily care, we recommend an early consultation with your supporter. |
| Neutral | Improvement | Also, let's keep moving in the right direction! |
| Neutral | Flat | Some adjustments may be needed to reach your target value. Please make use of consultations with your supporter. |
| Neutral | Deterioration | For better management of your daily care, we recommend an early consultation with your supporter. |
| Negative | Improvement | Also, let's keep moving in the right direction! |
| Negative | Flat | Some adjustments may be needed to reach your target value. Please make use of consultations with your supporter. |
| Negative | Deterioration | For better management of your daily care, we recommend an early consultation with your supporter. |

Fig. B2 illustrates GA value trends (left side) and system messages (right side), which were presented to a subject along with weekly changes of GA values actually measured for the subject. By using such a system, it is possible to generate an appropriate message regarding a GA value trend at each time and send the message to the subject.

<Example 2: Display on User Terminal>

Figs. C1 to C4 illustrate display examples of application software (commonly called "apuri" in Japanese, commonly called "App" in English, and also referred to as an "application" in this specification) on a user terminal 300. The user terminal 300 displays a screen that mimics a screen of a smartphone as available at the time of filing, but should not be interpreted as being limited thereto.

<Home Screen>

Fig. C1 illustrates an example of a layout of a home screen 310 of the user terminal 300. The home screen 310 displays a "goal of this week" 311 and a "change in GA value" 312.

The "goal of this week" 311 is a "goal" effective at that point in time. The initial goal is displayed for the first week, or, if the goal has been changed thereafter, a goal effective at that point in time is displayed. In some examples, the goal of the subject may be displayed on a home screen of an electronic terminal of the subject. Thus, each time the subject opens the application, the subject recognizes their own goal. Therefore, actions effective to prevent lifestyle-related diseases and motivation for behavior change can be maintained.

The "change in GA value" 312 displays a GA value trend. In Fig. C1, changes in GA value over 10 weeks are displayed. In some examples, latest and past GA values and/or a GA value trend may be displayed on the home screen of the electronic terminal of the subject. Thus, each time the subject opens the application, the subject recognizes the GA value trend. The subject can recognize how behaviors performed by the subject have affected the GA value. For example, when a "correct" behavior is performed and the GA value is improving, it becomes a reward for the subject. For example, when a certain behavior is performed and the GA value is deteriorating, it can be learned that the behavior adversely affects the change in GA value.

The home screen 310 further displays an "achievement status" 313. The "achievement status" 313 represents a frequency at which a GA value was measured and a frequency at which a diary (described below) was input at predetermined numbers of past timings. In this example, it is requested to measure a GA value and input a diary every week. Accordingly, the "achievement status" for the request is displayed.

The home screen 310 further displays some tools in a lower portion thereof (a dock or a toolbar). In Fig. C1, respective icons for jumping to a home screen 314, a message 315, and a calendar 316 are displayed. For example, in Fig. C1, the message 315 indicates that three messages have been sent from the system and are unread.

When pressing the message icon 315, the subject can check requests prompting each of output information from the system, messages, input of a diary (diary screen), and viewing of a new educational video (intermediate screens are not illustrated). These are described below.

When the subject presses the calendar icon 316, a calendar is displayed (not illustrated), and the subject can check past behavior-related information and GA values of the subject.

<Message Screen>

Fig. C2 illustrates an example of a layout of a message screen 320 of the user terminal 300. For example, a message 321 from the system may include a qualitative comment on a GA value trend, a correlation with behaviors that has been performed, and praise regarding any behavior that shows a good trend. The message (system message) 321 from the system may include a message indicating psychological support for the subject.

The subject can evaluate the received message 321 and input the evaluation. The message screen 320 of Fig. C2 displays "like"/"dislike" buttons 322. By pressing either, the subject can return to the system whether the message 321 was beneficial for the subject. The message screen 320 of Fig. C2 further displays a text input box 323. The subject can input their own comment (user message) regarding the received message 321. More detailed expressions or other information than by the like/dislike buttons 322 can be returned to the system. The system can refer to these evaluations by the subject and optimize an algorithm for generation of output information so as to enable provision of better support.

<Diary Screen>

Fig. C3 illustrates an example of a layout of a diary registration screen 330 of the user terminal 300. The subject inputs information regarding their own behaviors and presses a send button (336) on the diary registration screen 330, which can then be input to the system.

On the diary registration screen 330 of Fig. C3, the subject can input results of behaviors regarding diet 331, exercise 332, and medication 333 by a selection method in an easy manner. Regarding the diet 331 and the exercise 332, one can be selected from among three options, "goal achieved", "as usual", and "goal not achieved" by a button. Regarding the medication 333, one can be selected from among four options, "took all doses", "did not take some doses", "took none of my medication", and "no medication prescribed" by a button. Selection questions by button push make it easy for the subject to answer and can increase the frequency of diary input.

The diary registration screen 330 of Fig. C3 displays a text input box ("review") 334. The subject can return detailed expressions or other information to the system. In some embodiments, the "review" may be classified as a user message.

On the diary registration screen 330 of Fig. C3, body weight can also be recorded.

The system can refer to these diary inputs of the subject, namely, behavior-related information. Accordingly, for example, the system can optimize an algorithm for generation of output information so as to enable provision of better support. For example, the system can determine a psychological or physical risk to the subject together with a GA value trend.

<Video Screen>

Fig. C4 illustrates an example of a layout of a video screen 340 of the user terminal 300. The system sends an educational video considered appropriate to the subject. The subject can view a video 341 displayed on the video screen 340. The video screen 340 of Fig. C4 displays like/dislike/favorite buttons 342. By pressing any of them, the subject can return to the system whether the video 341 was beneficial for the subject. Further, when registered as a favorite, the video can be repeatedly viewed by the subject easily. The system can refer to these evaluations by the subject and optimize an algorithm for generation of output information so as to enable provision of better support.

<Example 3: Flowchart>

Fig. F1 illustrates a flowchart 9000 according to an example. In some embodiments, this flowchart may be used in, for example, the examples illustrated in Figs. C1 to C4. In some embodiments, this flowchart may be used in other examples or embodiments.

A subject starts use of an application (9001). If it is determined that the subject has not started use or that access to or registration with the application has been completed but substantial use has not been started, the system may prompt the subject to start use. The system may notify the subject via the application when communication via the application is possible. When the system cannot access the subject via the application, or even if communication via the application is possible, the system may contact the subject by a method without intervention of the application. For example, the system may send an email or a short message to the subject. For example, the system may notify a person in charge to contact the subject.

The subject sets an initial goal, for example, through human counseling (interview). The initial goal is input to the system. The system acquires the initial goal (9010). The system may acquire a medical history, constitution, a personality diagnosis result, and the like of the subject. The subject starts measurement of a GA value. This GA value is input to the system. The system acquires this initial GA value (9020).

In some embodiments, initial information may include a personality diagnosis result of the subject. Personality can affect behavioral change, improvement of lifestyle, how the subject considers their own behaviors and a GA value trend, maintenance of motivation, and the like. Depending on the personality type of the subject, processing performed by the system, such as analysis of input information, such as the content, styles, and timings of outputs to the subject, and optimization of various algorithms, and the like may be optimized.

After acquiring various pieces of initial information, the system starts a weekly cycle of the subject (9100). This cycle may correspond to a cycle of GA value measurement. A start day and an end day of a week may be set as appropriate. A cycle period need not be one week. Examples of other cycle periods include, without limitation, 5 days, 8 days, 10 days, 15 days, 2 weeks, 3 weeks, and a selected number of days or weeks of a life cycle of the subject. In some embodiments, a cycle period is a plurality of days and may be shorter than a cycle of HbA1c measurement.

The flowchart 9000 represents items performed within a cycle, which include processes regarding a new goal (9111, 9112), a weekly GA value and a GA value trend (9121, 9122), a diary record (9131, 9132), exchange of messages (9141, 9142), and viewing of an educational video (9151, 9152).

The subject may input a next-week goal every week. The system may acquire an input new goal of the subject (9111) and present the new goal to the subject (9112). Examples of timings at which a new goal is presented to the subject include output of a weekly GA value or a GA value trend (9122), a request for diary input or a diary input by the subject (9131, 9132), output or input of a message (9141, 9142), and viewing of a video by the subject (9151, 9152). The new goal may be presented to the subject at any other timing.

A GA value is basically or typically measured once a week. This weekly GA value is input to the system. The system acquires a GA value measured based on a body fluid acquired weekly by the subject (9121), generates a GA value trend including the value, and outputs the GA value trend to the subject (9122). Information on the GA value trend may include a comment. The system may use information on the comment to optimize a comment to be attached to a GA value trend that is output next (9340).

The system may request or prompt the subject to input a diary every day (9131). The subject may input selected answers to multiple-choice questions as illustrated in Fig. C3. The subject may input sentences to a "review" in response to sentence open-ended questions as illustrated in Fig. C4. When the subject inputs a diary, the system acquires the input information (9132). As the term implies, a diary is preferably written every day. However, there are cases where a diary is not input every day. The system may prompt diary input every day or at any other time interval (for example, politely remind if no diary input is made for several days), and may adjust the input timing. The system may optimize the algorithm based on the frequency, content, timing, and other characteristics of diary input (9340). A diary can be classified as behavior-related information.

The system may output a message to the subject independently or in answer to a question from the subject (9141). The message may be configured with information such as text, video, and voice, and the like. The subject may input an answer (reaction) to the message from the system. The system acquires this reaction (9142). The system may optimize a message to be next output to the subject, based on the reaction or overall content of communication, timings, and other characteristics (9340). The "message" to be output to the subject can be classified as beneficial information. The "reaction" of the subject can be classified as behavior-related information. These classifications are illustrative, and other classifications may be adopted.

The system may provide a video to the subject (9151). A video clip may include information useful for behavioral change of the subject, such as knowledge regarding diabetes mellitus and lifestyle and proposals. The system may select an appropriate video clip from among a large number of video clips prepared in advance, based on information regarding the subject (preferences, personality, knowledge, goals, a GA value trend, behavioral information, and the like). The subject may input an evaluation of the viewed video clip (9152).

The system may determine a risk that the subject stops use (drops out) of the system, based on a GA value trend, various pieces of input information from the subject, and/or various pieces of information regarding the subject, or a combination thereof (9210). The system may optimize each algorithm based on a result of dropout risk determination (9340). When it is determined that there is a risk that the subject will drop out, each algorithm may be optimized such that a probability of dropping out decreases (9340). Alternatively or simultaneously, it may be recommended that the subject receive human counseling (9310). In some embodiments, when it is determined that the dropout risk is high (9210), it may be recommended to receive human counseling (9310), and when there is a dropout risk to some extent but it is not so high, human counseling need not be recommended (9340). For example, even if there is a dropout risk, when it is low, each algorithm may be optimized (9340), and there is no need to recommend human counseling to the subject. In some embodiments, an algorithm for generating a message to be output to the subject (9141) may be optimized (9340).

When there is no dropout risk (9210), or, although not illustrated, even when there is a dropout risk, it may be determined whether there is another risk (9220). Examples of risks include, for example, the possibility of adverse events such as hypoglycemia and depression, or disease risks. In some embodiments, when it is determined that there is a disease risk or a possibility of an adverse event, the subject may be guided to human counseling (including medical practice) (9310). In some embodiments, when it is determined that a dropout risk is not avoidable through optimization of the system (not illustrated), the subject may be guided to human counseling.

In some embodiments, the system may provide information within the system about the subject to a counselor of human counseling. The counselor can acquire information on the subject and utilize the information for human counseling. The information related to the subject, such as a trend of GA values, the content of diary input, a self-set goal of the subject, and the viewing status of a video, or the like, for example, is beneficial for the counselor. Accordingly, the counselor can effectively perform human counseling and further can make human counseling more efficient.

A more human response can be provided to the subject. Thus, the content of human counseling can be utilized for optimization of each algorithm of the system. In some embodiments, the system may acquire the content of human counseling that has been performed. For example, the counselor may input the content of human counseling to the system. For example, the content of human counseling may be recorded via voice and video, and the system may acquire the information. Voice or video recordings may be organized by artificial intelligence.

In Fig. F1, when the dropout risk is low or there is no dropout risk (9210) or when there is no other risk (9220), that is, when the subject is not guided to human counseling, each algorithm is optimized (9340). However, the present disclosure is not limited thereto. Regardless of whether there is a dropout risk or any other risk (such as the possibility of an adverse event), or even when there is a dropout risk or any other risk, algorithms may be optimized. In Fig. F1, determination of a dropout risk (9210) and determination of any other risk (9220) are described as being performed sequentially; however, combinations of orders of these determinations should not be interpreted in a limiting manner. Determination of a dropout risk and determination of any other risk may be performed at any time during use of the system and may be performed in parallel.

When the possibility of an adverse event is recognized, it is preferable to immediately guide the subject to human counseling. On the other hand, in general, the incidence of a dropout risk is higher than the possibility of an adverse event. Accordingly, it is preferable to optimize each algorithm so as to reduce the rate of human counseling and also reduce the rate of dropout risk. As described above, with appropriate use of human counseling and efficient operation of application software, the system can be operated effectively and efficiently.

In some embodiments, while the subject basically uses the system, in predetermined cases, the system may provide an option in which the subject can use human counseling. Through a combination of the system involving no human intervention and human counseling, efficient and more effective support for glycemic management can be provided to the subject.

<Support for Initial Problems at Start of Use>

Guidance of the subject to human counseling can be performed based on various determinations in addition to the above. For example, a subject may stumble at the start of application use. The system recognizes that some initial event by the subject (such as execution of an agreement for use of the application, download of the application to an electronic terminal, or registration via the application) has occurred; however, thereafter, the system may recognize that the subject has not substantially started use of the application even after a predetermined time has elapsed or at a predetermined time. For example, the amount of time from one initial event to a next initial event may be longer than expected. In that case, the system may provide support to the subject in some way.

When substantial start of use of the application by the subject is not recognized, the status of the subject, such as forgetting a predetermined operation, lack of understanding of operation, or stopping use due to malfunction, may be inferred. The system may contact the subject or a person related to the subject in accordance with the inferred status. When the subject has not started use of the application at all and communication via the application is not possible, the system may send an email to the subject or send a short message to the subject if the subject's phone number is available, or may notify a person in charge to make a phone call. When communication via the application is possible, the system may communicate with the subject via the application. Accordingly, for example, the occurrence of initial problems can be avoided or reduced. When the subject still does not start use, the system may determine that human support is necessary and notify a person in charge to provide human support to the subject.

Input of a "review" in a "diary", and an answer to a "message" from the system or a spontaneous question or tweet ("reaction") are information regarding the subject, which is valuable and beneficial to the system.

Input from the subject is a kind of action performed by the subject and can indicate the level of motivation of the subject. By communicating with the system by themselves, the subject can form a virtual rapport with the system. The subject can recognize the system, which is a virtual existence, as a human existence. These recognitions of the subject are human psychological changes formed through communication such as human counseling and are considered to be beneficial for behavioral change and improvement of lifestyle. The system may determine a situation in which some input from the subject is being continued. Accordingly, the system may analyze or determine a relationship between changes in the GA value of the subject in a desirable direction or maintenance of the GA value at a desirable level and the status of behavioral change and improvement of lifestyle, changes in psychological state, or the like.

On the other hand, a decrease in the amount or frequency of input from the subject can also be a parameter indicating a change in the state of the subject. For example, when the subject has a personality that is not well-suited to communication, these pieces of input information can provide a limited opportunity to acquire the current state and changes of recognition of the subject. When there has been no input such as a "review" or a "reaction" from the subject for several weeks, the system may output a "message" asking the subject about their recent status. No input from the subject may indicate a possibility that the system is not being used favorably by the subject. In such a case, accordingly, the content, style, and timing of a "message" to be sent to the subject may be set to a restrained, gentle, and reduced-frequency mode rather than a proactive, bright, and frequent mode.

As described above, the system may predict a future GA value trend, behavior, state (including a psychological state), stage of behavioral change, and the like of a subject. In some embodiments, when those parameters of the subject differ from predicted ones, the system may optimize a communication style, output information, and the like. Alternatively, in some embodiments, when those parameters of the subject differ from predicted ones, the system may recommend human counseling to the subject or notify a person in charge to provide human support.

<Example 3: Results of Clinical Study>

This study targeted 99 subjects. Conditions for the subjects were that diagnostic criteria for type 2 diabetes mellitus by the Japan Diabetes Society were met, treatment with insulin or GLP-1 was not being received, an HbA1c value was 7.0 to 9.0% and there had been no recent change in therapeutic drugs, or the like and symptoms were stable, and consent to participate in the clinical study had been given. However, one subject who did not meet participation criteria of the study was excluded. The remaining 98 subjects were randomly assigned, according to a random number table, to an intervention group (GA and Mobile App) and a non-intervention group (Control). The two groups were compared at a single facility and in an open-label manner.

The subjects assigned to the intervention group sent, once a week, a minute amount of blood (capillary blood) specimen collected by self-fingerstick at home to a laboratory and received a GA test result via a smartphone app. Further, the subjects were asked to daily fill in an achievement status of goal behaviors, which was input in advance to the smartphone app, and were also allowed to revise a goal as needed. The non-intervention group received usual medical care by outpatient visits as before.

Of the assigned subjects, 93 subjects excluding those who no longer met participation criteria of the study during the study period or who withdrew consent to participate in the clinical study during the study, and the like (92 subjects at a point in time two months after the start of the clinical study) were targeted for analysis.

At the start of the study (Baseline) and on visit days one and two months after the start of intervention, venous blood sampling was performed in the intervention group (GA and Mobile App) and the non-intervention group (Control). A GA value was obtained by a usual test method. Fig. D1 illustrates a mean and a standard deviation of an amount of change (difference) from a GA value at the start of the study in each group, and a p-value when the two groups were compared by an unpaired two-sided t-test.

Fig. D2 illustrates a mean and a standard deviation of an amount of change of an HbA1c value obtained using the same blood specimen, a mean and a standard deviation of a change (difference) from an HbA1c value at the start of the study in the two groups, and a p-value when the two groups were compared by an unpaired two-sided t-test.

Figs. D3 to D5 respectively illustrate means and standard deviations of body weight changes (Fig. D3), BMI changes (Fig. D4), and abdominal circumference changes (Fig. D5) and results of an unpaired two-sided t-test at the start of the study (Baseline) and on visit days one and two months after the start of intervention between the intervention group (GA and Mobile App) and the non-intervention group (Control).

The subjects in the intervention group set goals and recorded their achievement by themselves via the smartphone app, and judged whether the goals were beneficial for glycemic management by weekly GA measurements and revised the goals. The subjects in the control group did not perform those. Results from Figs. D1 to D5 indicate that GA values and HbA1c values, which are indices of mean blood glucose levels, body weight, BMI, and abdominal circumference in the intervention group decreased statistically significantly compared to the control group. That is, weekly GA measurements and use of the smartphone app were shown to be useful for improvement of glycemic management and for decreases in body weight, BMI, and abdominal circumference. Furthermore, there were very few subjects who withdrew consent during the study. About 94% of the subjects completed participation in the study for two months. Therefore, the method was determined to have a low burden on subjects and/or to have high feasibility.

<Reward Day>

In some embodiments, as a self-evaluation of the day or the week, the system may provide a subject with an option of a "reward day" or a "cheat day" (in this specification, the two terms are used with the same meaning and interchangeably). The above example has described a question with three choices such as "well done", "average", and "failed". In some embodiments, in place of the "failed" choice, a "reward day" may be provided. Alternatively, a selection question including a "reward day" may be set. That is, in place of or in addition to a self-evaluation of "failure to execute the goal behavior", a "reward day" choice may be provided.

In many cases, a "reward day" in management of lifestyle is set in advance for a certain day in the near future. In some embodiments of the present application, a "reward day" may be set similarly. On the other hand, in some embodiments of the present application, a "reward day" may be set as a post hoc self-evaluation option for a certain day or week.

In data of a clinical study conducted by the present applicant, results were obtained, indicating that subjects who selected a "reward day", which was set to indicate the failure to actually execute the goal behavior, showed a more effective decrease in GA value than subjects who selected "as usual" (not described).

Although this psychological or medical interpretation should not be made in a limiting manner, one possible interpretation is as follows. That is, when a fact that a goal behavior was not achieved well is evaluated as "failed", negative emotions may be generated and ingrained in the subject's mind. On the other hand, there is a possibility that, based on the same fact, that day may be retrospectively reinterpreted as a "reward day". Reducing negative emotions while accepting facts as facts may lead to more effective behavioral change or glycemic management.

In some embodiments, a subject who routinely selects a "reward day" may be evaluated as not having high motivation for behavioral change. In some embodiments, a subject who selects a "reward day" at a predetermined frequency (for example, a frequency of twice or three times or more per week) may be evaluated as not properly conducting a self-evaluation or as having high motivation for behavioral change. For example, when a subject repeatedly selects both "satisfied that the goal behavior has been executed successfully" and a "reward day", or selects both at a predetermined frequency, the subject may be evaluated as consciously conducting a self-evaluation, having high literacy, or having high motivation for behavioral change.

In some embodiments, when a low evaluation regarding selection of a "reward day" continues for a predetermined period of time, the system may change a communication style or may notify the subject that human counseling should be received.

<Example: Configuration and Operation of System>

Fig. E1 schematically illustrates a network environment connecting between a system 100 and a subject 200 according to an embodiment of the present disclosure.

The system 100 includes a central processing unit (CPU, also referred to herein as a "processor" and a "computer processor") 101, a storage unit (storage) 102, a main memory or a memory location 103, a peripheral device 104, and a communication interface 105. These components communicate with the CPU 101 via a communication bus 106 such as a motherboard.

The CPU 101 may be a single-core or multicore processor or a plurality of processors for parallel processing. The CPU 101 may include a computing architecture that processes data signals and implements various architectures or combinations of instruction sets. The CPU has a cache (not illustrated) inside, and the cache can communicate data at high speed with a registry (not illustrated) of the CPU 101. The cache stores data to be used by the CPU 101, and the CPU 101 can process the data at high speed. Access to the cache is not specified below.

The storage unit or storage 102 is a non-transitory storage medium that stores data for providing the functions described herein. The storage unit 102 may be a data storage unit (or data repository) for storing data. The storage unit 102 stores software, programs, and data that can be used by the system 100. Examples of the storage unit 102 include, without limitation, dynamic random access memory (DRAM), static random access memory (SRAM), flash memory, and other memory devices. Other examples of the storage unit 102 include, without limitation, a hard disk, a floppy disk, a CD-ROM, a DVD-ROM, a DVD-RAM, a DVD-RW, flash memory, a magnetic tape, and any other mass storage device for storing information on a persistent basis, a nonvolatile memory, or similar persistent storage devices and media.

The main memory 103 can store instructions or data executable by the CPU 101. Nonlimiting examples include dynamic random access memory (DRAM), static random access memory (SRAM), read-only memory (ROM), and flash memory. The CPU 101 can move software, programs, and data stored in the storage unit 102 to the main memory 103 and execute and use the software, programs, and data.

The peripheral device 104 may be, for example without limitation, other memory, data storage, and/or an electronic display adapter or the like. The communication interface 105 may be, for example without limitation, a network adapter or the like. The communication interface 105 can communicate with other equipment via a network 400.

There can be a plurality of modes in which the subject 200 acquires a GA value and provides the GA value to the system 100.

In some embodiments, the subject 200 can take a mail-in test. The subject 200 may collect their own body fluid (such as blood or saliva) using a predetermined mail-in kit 211 and send the collected body fluid to a test center 220. The test center 220 can measure a GA value of the subject 200 using the received body fluid of the subject 200. The test center 220 can communicate with the system 100 via a communication interface 221 and the network 400. The test center 220 provides the acquired GA value of the subject 200 to the system 100.

In some embodiments, the subject 200 may visit a medical institution 230. The subject 200 may have their own body fluid collected and a GA value measured at the medical institution 230. The medical institution 230 can measure a GA value of the subject 200 using the received body fluid of the subject 200. The medical institution 230 can communicate with the system 100 via a communication interface 231 and the network 400. The medical institution 230 provides the acquired GA value of the subject 200 to the system 100.

In some embodiments, the subject 200 may measure a GA value using a GA measuring apparatus 240 for self-monitoring (or home use). The subject 200 may introduce their own body fluid into the measuring equipment 240 and have a GA value measured with the measuring equipment 240. The measuring equipment 240 can communicate with the system 100 via an internal or externally attached communication interface (not illustrated) and the network 400. The measuring equipment 240 provides the acquired GA value of the subject 200 to the system 100.

The GA value of the subject 200, which is acquired by the illustrated or non-illustrated mode, is transmitted to the computer system 100 via the network 400, together with auxiliary information (such as an ID of the subject 200, a date and time of body fluid collection, and information related to the measurement, or the like), and is received by the communication interface 105. The information received by the communication interface 105 is temporarily stored in the main memory 103 and is moved to and stored in the storage 102 as appropriate.

The subject 200 has an electronic terminal 300 capable of communication (for example, a smartphone, a tablet device, a personal computer (PC), or the like). The electronic terminal 300 is configured to be connectable to or communicable with the network 400.

The subject 200 can start, change, stop, or cancel a contract for use of the system 100 via the electronic terminal 300. The system 100 can establish or implement communication only with an authorized or contractually effective subject 200 or the electronic terminal 300 of the subject 200 in accordance with personal information, copyright law, civil law, and other laws and in, furthermore, an ethically and medically appropriate manner. The subject 200 can communicate with the system 100 via the electronic terminal 300 as a legitimate user of the system 100.

The subject 200 collects a body fluid every week or has a body fluid collected every week. A GA value is measured therefrom and transmitted to the system 100. The system 100 transmits the received GA value to the electronic terminal 300 of the subject 200. Accordingly, the subject 200 can learn their own GA value via the electronic terminal 300. When using the GA measuring apparatus 240 for self-monitoring, the subject 200 can learn their own GA value on a display thereof or on the electronic terminal 300 connected to the GA measuring apparatus 240 for self-monitoring.

As the subject 200 collects their own body fluid and measures a GA value every week, information on GA values is accumulated in the storage 102. The CPU 101 generates a GA value trend based on the latest GA value and past GA values and transmits the GA value trend from the communication interface 105. The GA value trend is transmitted to the electronic terminal 300 of the subject 200 via the network 400. Accordingly, the subject 200 can learn their own GA value trend via the electronic terminal 300.

The subject 200 can cause the system 100 to store their own goal. In some embodiments, the goal of the subject 200 may be input to the system 100 by any other person such as a healthcare professional or a counselor. In some embodiments, the goal of the subject 200 may be input to the electronic terminal 300 by the subject 200 and transmitted to the system 100 via the network 400. The system 100 stores information including the received goal of the subject 200 in the storage 102.

The system 100 may transmit, together with the GA values, information including the goal of the same subject 200, which is stored in the storage 102, to the electronic terminal 300. The system 100 can transmit the information including the goal of the subject 200 to the electronic terminal 300 at any other timing.

The subject 200 can input their own behavior-related information to the electronic terminal 300. The electronic terminal 300 transmits the behavior-related information of the subject 200 to the system 100 via the network 400. The system 100 stores the received behavior-related information of the subject 200 in the storage 102.

The storage 102 stores a large number of educational videos. The CPU 101 refers to the goal of the subject 200, the GA values, the GA value trend, the inputs from the subject 200, other information, and the like, and selects an educational video to be provided to the subject 200. The system 100 transmits the selected educational video from the communication interface 105 to the electronic terminal 300 of the subject 200.

The CPU 101 refers to the goal of the subject 200, the GA values, the GA value trend, the inputs from the subject 200, other information, and the like, and generates a message to be provided to the subject 200. The system 100 transmits the generated message from the communication interface 105 to the electronic terminal 300 of the subject 200.

The CPU 101 stores the generated data, the content of information transmitted to the subject 200, the time, and other logs in the storage unit 102.

The subject 200 can view, listen to, and read the educational information and message received by the electronic terminal 300. The subject 200 can input an evaluation regarding the received information to the electronic terminal 300. The electronic terminal 300 transmits the input evaluation by the subject 200 to the system 100. The system 100 stores the received evaluation by the subject 200 in the storage 102.

The CPU 101 can analyze the GA values and inputs of the subject 200, namely, input information such as the GA values, the GA value trend, the goal, behavioral information, and the evaluation.

The CPU 101 may refer to the goal of the subject 200, the GA value trend of the subject 200, the behavioral information of the subject 200, and/or the evaluation by the subject 200 to optimize an algorithm for proposing and transmitting a goal, an algorithm for selecting and transmitting educational information, an algorithm for generating and transmitting a message, and the like. With the optimization of the content of the goal to be proposed, the content of the educational information, the content of the message, timings at which these are provided to the subject 200, and the like, support to be provided by the system 100 for the subject 200 can be better matched to individuality, behavioral modes, psychological states, and changes therein of the subject 200. Accordingly, for example, the motivation of the subject 200 can be maintained or improved, or the risk of losing motivation can be reduced. For example, biomarker values (GA value, HbAlc value, BMI, body weight, blood pressure, and others) of the subject 200 can be improved and deterioration thereof can be avoided or reduced. For example, a lifestyle-related disease (such as diabetes mellitus) of the subject 200 can be improved, or deterioration thereof can be avoided or reduced. For example, health of the subject 200 can be promoted.

The CPU 101 may refer to the goal of the subject 200, the GA value trend of the subject 200, the behavioral information of the subject 200, and/or the evaluation by the subject 200 to determine whether the subject 200 has or may have a disease risk. For example, based on these pieces of information, the CPU 101 may determine whether the subject 200 is or may be in a state of hypoglycemia.

When it is determined that there is a possibility that the subject 200 has a risk, the CPU 101 may transmit to the subject 200 a message indicating that the subject 200 should receive human counseling or a diagnosis by a physician. The CPU 101 may simultaneously or separately transmit a message indicating that the system 100 does not assume any responsibility regarding the health or disease of the subject 200. The subject 200 receives the message on the electronic terminal 300 and can determine at their own responsibility whether to receive human counseling or a diagnosis by a physician.

The subject 200 may receive human counseling at their own request or due to a change in the contract for use. The subject 200 requests this from the system 100 via the electronic terminal 300. The system 100 can permit the subject 200 to access human counseling, upon confirmation of consistency of the request of the subject 200 with the contract or confirmation of a change in the contract. Human counseling may be an option or a premium service.

The subject 200 can have an interview with a counselor 500 in charge. The counselor 500 has an electronic terminal 510. The electronic terminal 510 can communicate with the system 100 via the network 400. The system 100 transmits information regarding the subject 200, namely, information such as the GA values, the GA value trend, the behavior-related information, and the evaluation, to the electronic terminal 510 of the counselor 500. Sharing these pieces of information with the counselor 500 enables efficient and/or effective human counseling for the subject 200.

The CPU 101 may obtain a customer retention rate, based on the number of subjects 200, the number of active subjects, the number of subjects who have switched to human counseling, and the like.

The CPU 101 may use the customer retention rate as an evaluation index to optimize an algorithm for proposing and transmitting a goal, an algorithm for selecting and transmitting educational information, an algorithm for generating and transmitting a message, and the like.

<Example: Functional Configuration of System>

Fig. E2 illustrates components in a functional perspective (functional components) of the system 100 according to an embodiment of the present disclosure. These functional components need not necessarily correspond to physical devices, parts, units, or the like of electronic equipment. Each functional component may be configured with one or more physical devices, parts, or units of electronic equipment, or a portion thereof, or a combination thereof. The expression "... section" as used herein may be replaced with "... means" or "... unit".

The system 100 includes a communication section 110, a control section 120, and a storage section 130.

The communication section 110 may be configured to include, for example without limitation, a processor that executes software, a communication I/F, and the like. The communication section 110 includes a receiving section 111 having a function of receiving data from the network 400 and a transmitting section 112 having a function of transmitting data to the network 400. The communication section 110 has a function of connecting to the network 400 and communicating with equipment (also referred to as GA measuring equipment) 221, 231, and 240 for measuring or retaining GA values, the user electronic terminal 300, and the counselor terminal 510.

The receiving section 111 can receive GA value-related information transmitted from the GA measuring equipment 221, 231, and 240, and a goal, an evaluation, and other input information transmitted from the user terminal 300. The transmitting section 112 can transmit output information generated by the control section 120 to a predetermined terminal via the network 400.

The storage section 130 may be configured to include, for example without limitation, a storage area of a storage medium. The storage section 130 can store and accumulate data (GA value data) 131 regarding GA values transmitted from the GA measuring equipment 221, 231, and 240. The storage section 130 can store and accumulate data (goal data) 132 regarding a goal of the subject 200, which is transmitted from the user electronic terminal 300, information (behavioral information data) 133 related to behaviors of the subject, and data (evaluation data) 134 regarding an evaluation of an output by the subject 200. The storage section 130 stores algorithms 135 to be used by the control section 120.

The control section 120 may be configured to include, for example without limitation, a processor that executes software code or programs. In some embodiments, the control section 120 may include a goal generation section 121 and a GA value trend generation section 122. The control section 120 may further include any one, at least one, a plurality, or all of a beneficial information generation section 123, an input information analysis section 124, an optimization section 125, a risk determination section 126, and a customer retention rate determination section 127.

The goal generation section 121 has a function of generating a goal of a subject or a goal to be proposed to the subject with reference to the input information and outputting the generated goal to the subject. The GA value trend generation section 122 has a function of generating a GA value trend based on acquired GA values and, optionally, with reference to the input information, and outputting the generated GA value trend to the subject. The beneficial information generation section 123 has a function of generating information to be output to the subject, such as an educational video and a message, optionally, with reference to the input information, and outputting the generated information to the subject. The input information analysis section 124 has a function of analyzing the input information. The optimization section 125 has a function of optimizing algorithms to be executed by the control section 120 with reference to the input information, analysis results thereof, results of risk determination, a customer retention rate, and the like. The risk determination section 126 has a function of determining whether there is a probability that the subject has a risk, with reference to the GA value trend, the input information from the subject, and/or input information analysis results or the like. The customer retention rate determination section 127 has a function of obtaining or determining a retention rate of system users (subjects).

The present disclosure includes the following embodiments:

A001. A method for assisting glycemic control of a subject using a computer system, the method comprising:

    a) acquiring a self-set goal of the subject;
    b) acquiring a GA value of the subject;
    c) generating a GA value trend using a plurality of the GA values;
    d) outputting the GA value trend to the subject; and
    e) outputting the self-set goal of the subject to the subject.

A002. The method according to A001 or any embodiment, wherein

    d) outputting the GA value trend to the subject and e) outputting the self-set goal of the subject to the subject are executed substantially simultaneously, or
    the GA value trend and the self-set goal of the subject are output to the subject so as to be recognizable by the subject substantially simultaneously.

A005. The method according to A001, A002, or any embodiment, wherein
b) acquiring a GA value of the subject includes acquiring a weekly GA value of the subject.
A011. The method according to any one of A001 to A005 or any embodiment, further comprising:

    requesting the subject to input information related to a behavior of the subject (behavior-related information) to the computer system; and
    providing an option to the subject to allow the subject to input information related to a behavior of the subject (behavior-related information) to the computer system.

A012. The method according to A001, A002, or any embodiment, further comprising:
requesting the subject to input information related to a behavior of the subject (behavior-related information) to the computer system (so as to input a diary) daily, weekly, during a predetermined period of time, or without specifying a period of time.

A013. The method according to A012 or any embodiment, wherein

the self-set goal of the subject includes a goal related to diet, exercise, and medication, and
the behavior-related information includes a degree of achievement of the goal of the diet, exercise, and medication.

A014. The method according to A013 or any embodiment, wherein
the degree of achievement of the goal of the diet, exercise, and medication is configured to be input as an answer to a multiple-choice question.

A015. The method according to any one of A011 to A014 or any embodiment, wherein
the behavior-related information is configured to be input as a free answer to an open-ended question.

A018. The method according to any one of A011 to A015 or any embodiment, comprising:
acquiring the behavior-related information.

A021. The method according to A001, A002, or any embodiment, comprising:

generating output information for the subject, based on at least part of the goal and the GA value trend; and
outputting the output information to the subject.

A021b. The method according to A001, A002, or any embodiment, comprising:

generating a message (system message) for the subject, based on at least part of the goal and the GA value trend; and
outputting the system message to the subject.

A022. The method according to any one of A001 to A015 or any embodiment, comprising:

adjusting or determining a communication style for the subject, based on input information from the subject (including at least part, a plurality, or all of the goal, the GA value trend, a state, a psychological state, knowledge, and a stage of behavioral change) or information related to the subject, and generating output information for the subject; and
outputting the output information according to the communication style to the subject.

A022b. The method according to any one of A011 to A015 or any embodiment, comprising:

generating a message (system message) for the subject, based on at least part of the goal, the GA value trend, and the behavior-related information; and
outputting the system message (as output information) to the subject.

A031. The method according to A001, A002, or any embodiment, comprising:
providing an input field for the subject to input a message (user message) for a system.

A032. The method according to A031 or any embodiment, wherein
the computer system is configured to allow the subject to communicate with the computer system via the user message and the system message.

A033. The method according to A032 or any embodiment, wherein
the computer system includes a chatbot or a communication AI for the subject to communicate with the computer system.

A035. The method according to A031, A032, or any embodiment, (further comprising: acquiring behavior-related information of the subject; and
acquiring the user message of the subject for the system,)
comprising:

generating a message (system message) for the subject, based on at least part of the goal, the GA value trend, the behavior-related information, and the user message; and
outputting the system message (as output information) to the subject.

A041. The method according to A031, A032, or any embodiment, (further comprising: acquiring behavior-related information of the subject; and

acquiring the user message of the subject for the system,) comprising:

generating a goal to be proposed to the subject, based on at least part of the goal, the GA value trend, the behavior-related information, and the user message; and
outputting the proposed goal (as output information) to the subject.

A051. The method according to any one of A001 to A041 or any embodiment, further comprising:
providing educational information (as output information) to the subject.
A052. The method according to A051, A052, or any embodiment, wherein
the computer system is configured to allow the subject to input an evaluation regarding the educational information, or an option is provided to allow the subject to input an evaluation regarding the educational information to the computer system.
A055. The method according to A051 or A052 and/or any embodiment, wherein
the providing of the educational information includes selecting the educational information based on at least part of the goal, the GA value trend, the behavior-related information, the user message, and an evaluation regarding the educational information.
A061. The method according to any one of A001 to A055 or any embodiment, further comprising:
(acquiring behavior-related information of the subject;

acquiring a user message of the subject for a system;
outputting a system message to the subject;
providing educational information to the subject; and)
acquiring an evaluation by the subject regarding information output or provided to the subject.

A062. The method according to A061 or any embodiment, wherein
the evaluation by the subject includes at least one or a plurality of:

an evaluation regarding the GA value trend;
an evaluation regarding the educational information; and
an evaluation regarding the system message.

A063. The method according to A061, A062, or any embodiment, wherein
at least one or a plurality of:

generating a new goal;
selecting the educational information; and
generating the system message

are performed based on the evaluation by the subject.
A065. The method according to any one of A001 to A063 or any embodiment, further comprising:
analyzing information (input information) input from the subject.
A071. The method according to any one of A011 to A065 or any embodiment, further comprising:
based on at least part of the input information and/or analysis of the input information, optimizing at least one or a plurality of:

an algorithm for generating the new goal;
an algorithm for selecting the educational information; and
an algorithm for generating the message.

A072. The method according to A071 or any embodiment, wherein
the optimizing is performed using artificial intelligence.
A075. The method according to any one of A001 to A072 or any embodiment, comprising:

inferring a state of the subject, based on at least part or all of the goal set by the self-set subject, the GA value trend, input information from the subject, and information regarding the subject;

determining, based on the state of the subject, a communication style suitable for communication with the subject;

generating output information, based on the state of the subject and the communication style; and

outputting the output information to the subject in accordance with the communication style.

A076. The method according to A075 or any embodiment, wherein

the input information from the subject or the information regarding the subject includes at least part of a diary, a self-evaluation, a like, an answer to a medical interview question, data from a wearable device, and health-related data.

A077. The method according to A0075, A0076, or any embodiment, wherein

the state of the subject includes at least part of a psychological state, knowledge, and a stage of behavioral change of the subject.

A078. The method according to A0075 or any embodiment, wherein

the output information includes a goal behavior to be proposed to the subject.

A081. The method according to any one of A001 to A076 or any embodiment, further comprising:

determining whether the subject has a risk, based on one or a plurality of pieces of the input information and/or the input information (analysis thereof).

A082. The method according to A081 or any embodiment, wherein

the risk includes a possibility of an adverse event, a disease risk, and/or a risk of stopping or suspending use of the computer system.

A083. The method according to A081, A082, or any embodiment, further comprising:

recommending human counseling to the subject when it is determined that the subject has the risk.

A085. The method according to any one of A001 to A083 or any embodiment, further comprising:

determining that human support is necessary for the subject when it is determined that the subject encounters an initial problem.

A091. The method according to any one of A001 to A085 or any embodiment, further comprising:

acquiring a customer retention rate.

A092. The method according to any one of A071, A072, and A091 or any embodiment, wherein

the optimizing of the algorithm or algorithms includes optimizing the algorithm or algorithms based on the customer retention rate.

B001. A method for assisting glycemic control of a subject using a computer system,

the computer system comprising:

a processor;

a communication unit connected to the processor and capable of communicating with an electronic terminal accessible by the subject; and

a memory connected to the processor,

the method comprising:

the processor acquiring, via the communication unit, a self-set goal set of the subject and storing the goal in the memory;

the processor acquiring, via the communication unit, a GA value of the subject and storing the GA value in the memory;

the processor generating a GA value trend with reference to a plurality of the GA values stored in the memory;

the processor transmitting, via the communication unit, the GA value trend to the electronic terminal of the subject and causing the electronic terminal to output the GA value trend to the subject; and

the processor transmitting, via the communication unit, the self-set goal of the subject to the electronic terminal of the subject and causing the electronic terminal to output the goal to the subject.

B002. The method according to B001 or any embodiment, wherein

the GA value trend and the self-set goal of the subject are output to the subject so as to be recognizable by the subject substantially simultaneously.

B011. A method for assisting glycemic control of a subject using a computer system,

the computer system comprising:

a processor;

a communication unit connected to the processor and capable of communicating with an electronic terminal accessible by the subject; and
a memory connected to the processor,

the method comprising causing the processor to execute the method and/or steps according to any one of A001 to A092 or any embodiment.

C001. Software comprising computer-executable code for assisting glycemic management of a subject, the computer-executable code being configured to, when executed on a processor,
cause the processor to:

acquire, via a communication unit, a self-set goal of the subject and store the goal in a memory;
acquire, via the communication unit, a GA value of the subject and store the GA value in the memory;
generate a GA value trend with reference to a plurality of the GA values stored in the memory;
transmit, via the communication unit, the GA value trend to the electronic terminal of the subject and cause the electronic terminal to output the GA value trend to the subject; and
transmit, via the communication unit, the goal to the electronic terminal of the subject and cause the electronic terminal to output the self-set goal of the subject to the subject.

C011. Software comprising computer-executable code for assisting glycemic management of a subject, the computer-executable code being configured to, when executed on a processor,
cause the processor to:
execute the method and/or steps according to any one of A001 to A092 or any embodiment via a communication unit and an electronic terminal of the subject.

D001. A non-transitory storage medium storing the software according to C001, C011, or any embodiment.

E001. A computer system for assisting glycemic control of a subject,
the computer system comprising:

a processor;
a communication unit connected to the processor and capable of communicating with an electronic terminal accessible by the subject; and
a memory connected to the processor, wherein
the processor is programmed to:

acquire, via the communication unit, a self-set goal of the subject and store the goal in the memory;
acquire, via the communication unit, a GA value of the subject and store the GA value in the memory;
generate a GA value trend with reference to a plurality of the GA values stored in the memory;
transmit, via the communication unit, the GA value trend to the electronic terminal of the subject and cause the electronic terminal to output the GA value trend to the subject; and
transmit, via the communication unit, the self-set goal set of the subject to the electronic terminal of the subject and cause the electronic terminal to output the self-set goal of the subject to the subject.

E005. The computer system according to E001 or any embodiment, wherein
the GA value trend and the self-set goal of the subject are output to the subject so as to be recognizable by the subject substantially simultaneously.

E006. The computer system according to E001 or any embodiment, wherein
the processor is further programmed to:

infer a state of the subject, based on at least part or all of the self-set goal of the subject, the GA value trend, input information from the subject, and information regarding the subject;
determine a communication style with the subject, based on the state of the subject;
generate output information, based on the state of the subject and the communication style; and
output the output information to the subject in accordance with the communication style.

E007. The computer system according to E006 or any embodiment, wherein
the state of the subject includes at least part of a psychological state, knowledge, and a stage of behavioral change of the subject.

E008. The computer system according to E006 or any embodiment, wherein

the output information includes a goal behavior to be proposed to the subject.

E011. A computer system for assisting glycemic control of a subject,

the computer system comprising:

a processor;
a communication unit connected to the processor and capable of communicating with an electronic terminal accessible by the subject; and
a memory connected to the processor, wherein

the processor is programmed to execute the method and/or steps according to any one of A001 to A092 or any embodiment.

E021. A computer system for assisting glycemic control of a subject, the computer system comprising:

a processor;
a communication unit capable of communicating with an electronic terminal of the subject;
a memory; and
a non-transitory storage medium storing the software according to C001, C011, or any embodiment.

E101. A computer system for assisting glycemic control of a subject, the computer system comprising:

receiving means for acquiring a self-set goal of the subject and a GA value of the subject;
storage means for storing the received GA value and goal;
GA value trend generation means for generating a GA value trend, based on a plurality of the GA values stored in the storage means; and
transmitting means for transmitting the self-set goal of the subject and the GA value trend to a terminal of the subject.

E102. The computer system according to E101 or any embodiment, wherein
the receiving means acquires at least one or a plurality of behavior-related information, a user message, and an evaluation.

E111. The computer system according to E101, E102, or any embodiment, further comprising:

message generation means for generating a system message;
educational information selection means for selecting educational information; and
at least one or a plurality of:

analysis means for analyzing input information from the subject, the input information having been received; and
optimization means for optimizing at least one or a plurality of algorithms for generating output information to be output to the subject, based on the input information from the subject.

While preferred embodiments of the present invention have been illustrated and described herein, it will be apparent to a person skilled in the art that such embodiments are provided only as examples. The present invention is not intended to be limited by specific examples provided herein. Any of the embodiments or any aspect disclosed herein is independently combinable, partly or wholly, with other embodiments and aspects described herein in any manner. For example, one, two, or three or more embodiments may be combinable in whole or in part.

A group of items connected by the conjunction "and" should not be read as requiring that all of those items be present in the group, and should be read as "and/or" unless otherwise specifically indicated. Similarly, a group of items connected by the conjunction "or" should not be read as requiring mutual exclusivity among the items of the group, and should be read as "and/or" unless otherwise specifically indicated.

While the present invention has been described with reference to the above specification, the descriptions and drawings of the embodiments in this specification are not intended to be construed in a limiting sense. Numerous variations, changes, and substitutions will occur to a person skilled in the art without departing from the present invention. Furthermore, it is to be understood that all aspects of the present invention are not limited to specific depictions, configurations, or relative proportions described herein that depend on various conditions and variables. It is to be understood that various alternatives to the embodiments of the present invention described herein may be used in practicing the present invention. Accordingly, the present invention is considered to encompass such alternative embodiments, modified embodiments, variant embodiments, or equivalents. The following claims define the scope of the present invention, and it is intended that methods and structures and their equivalents within these claims be covered thereby.

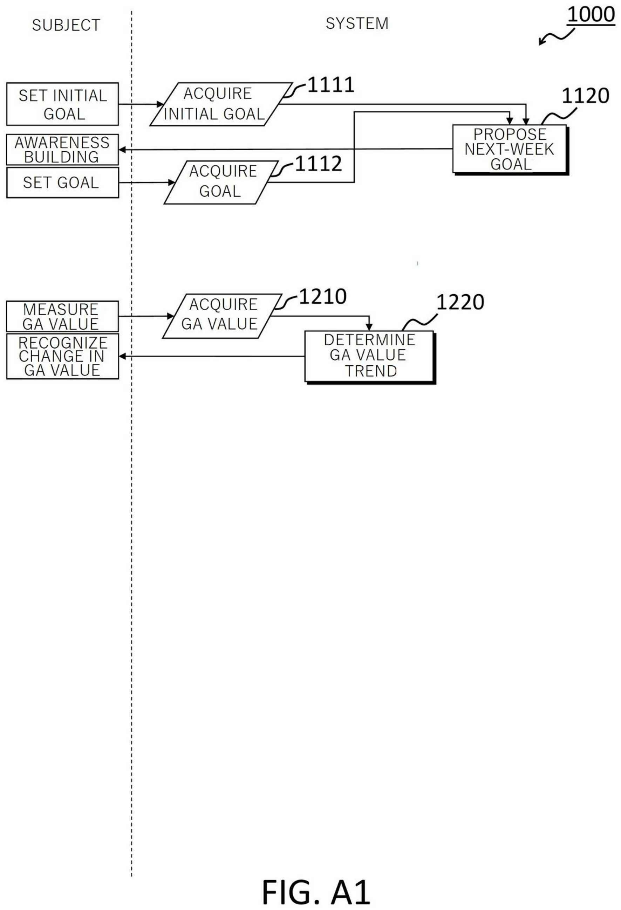

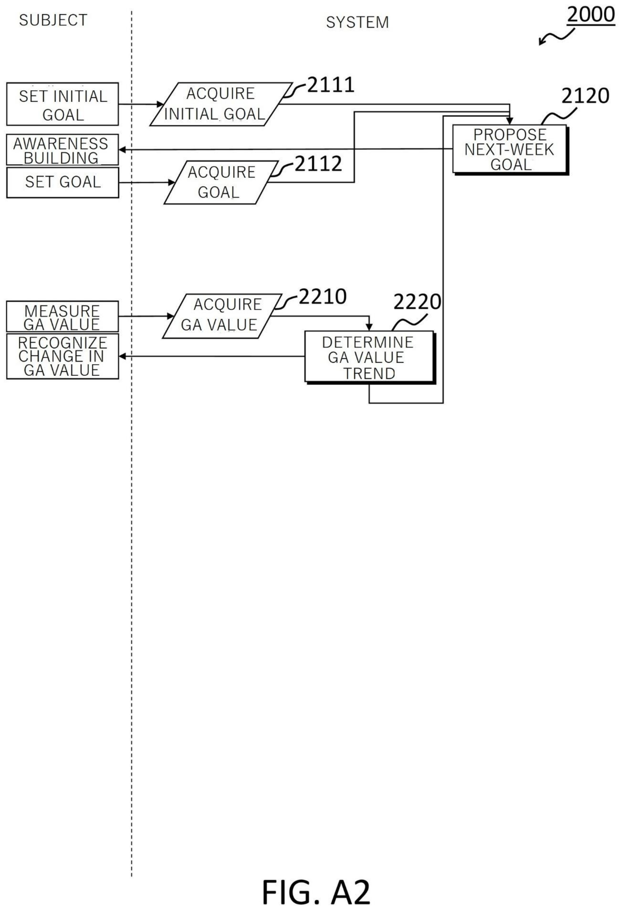

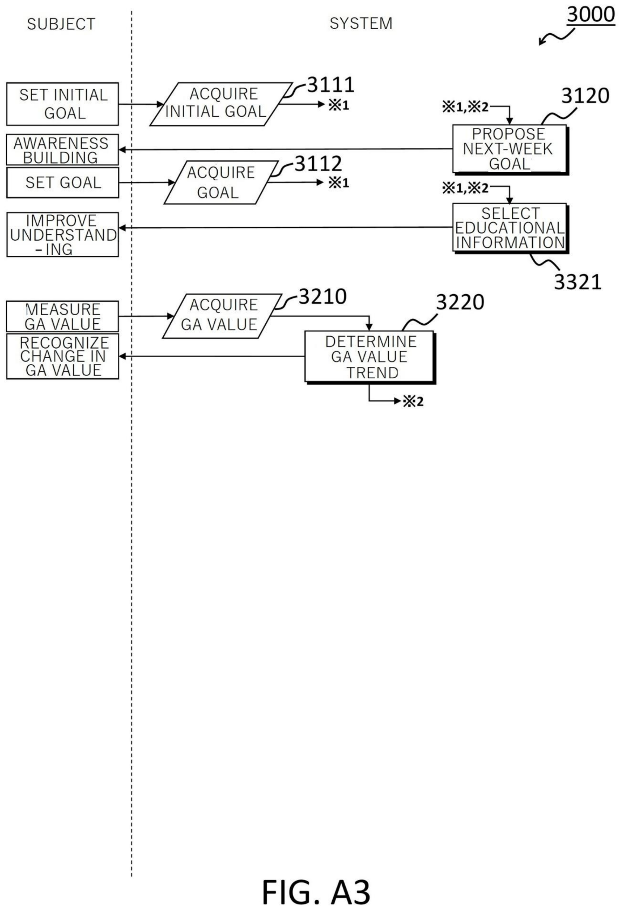

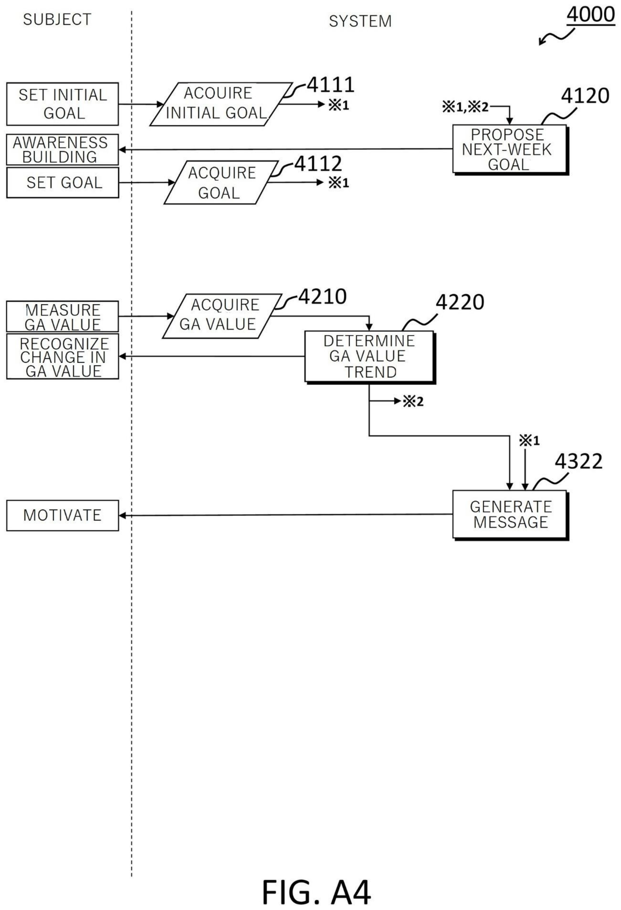

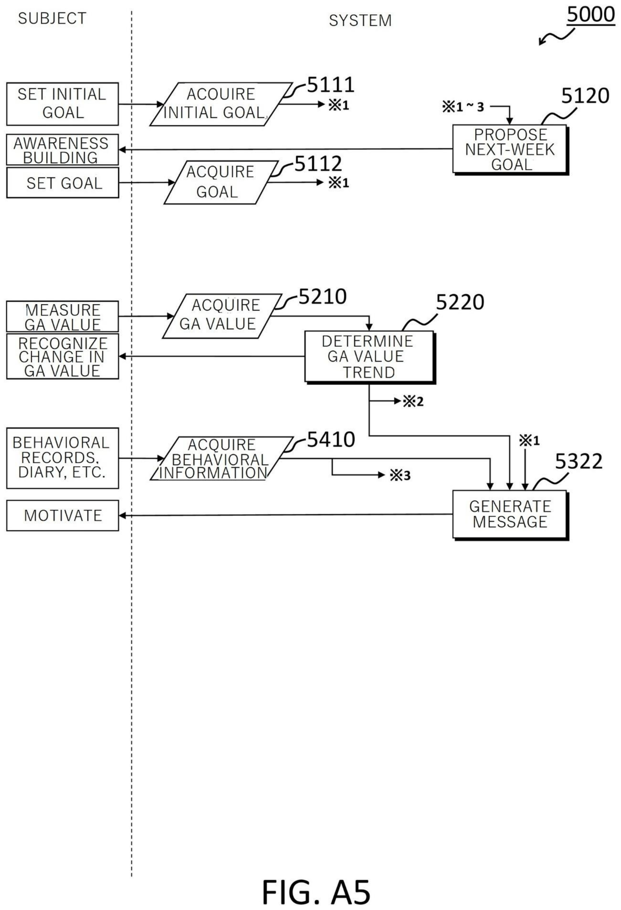

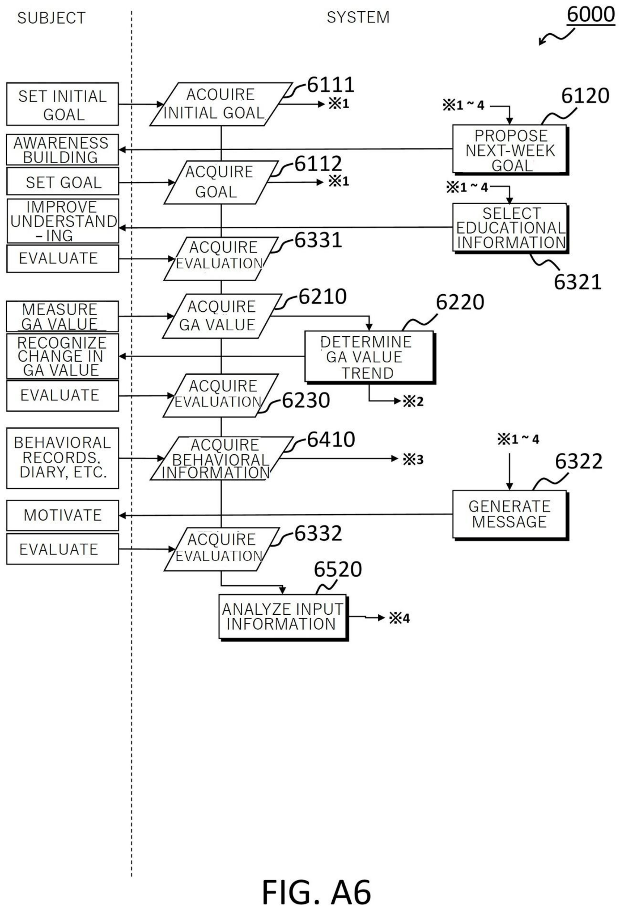

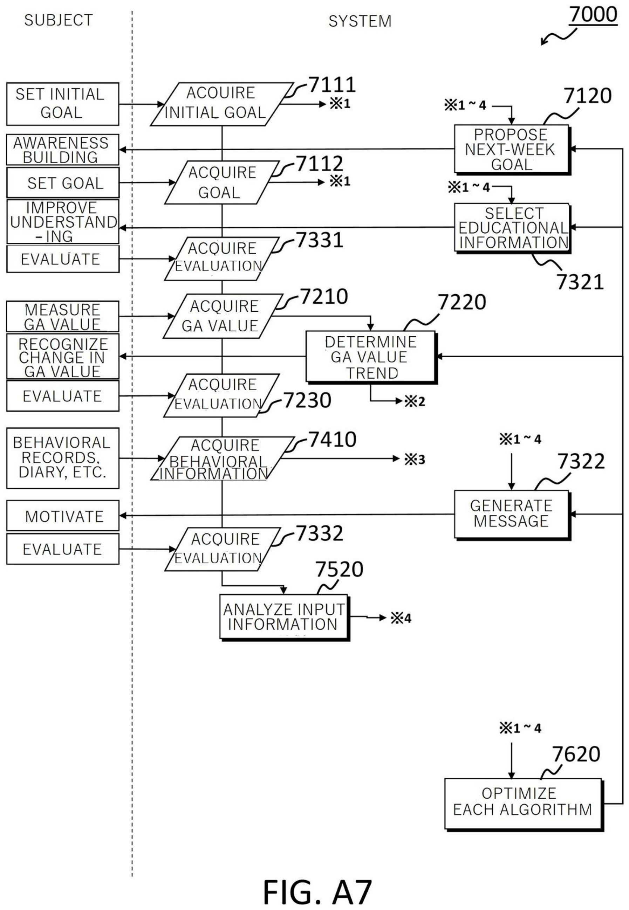

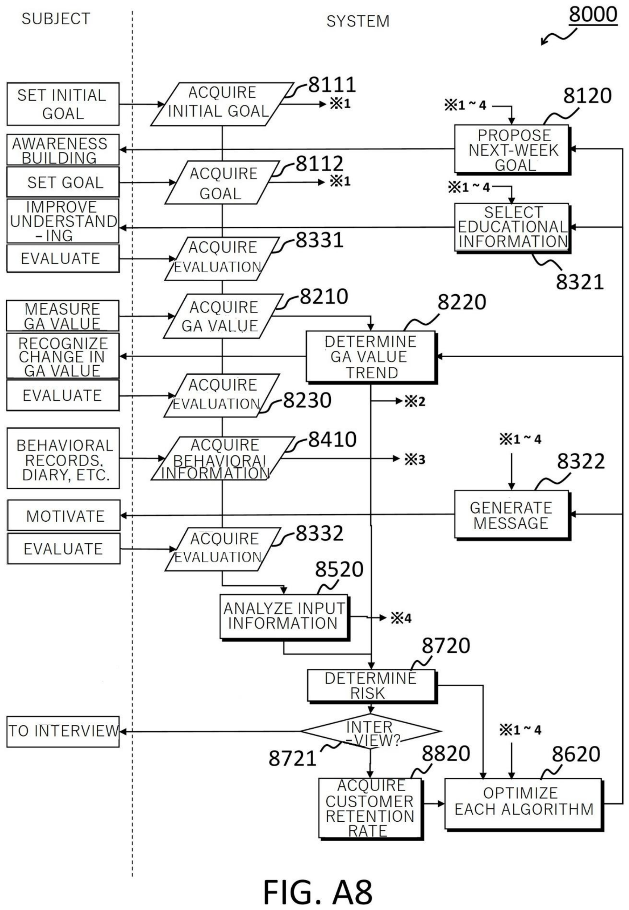

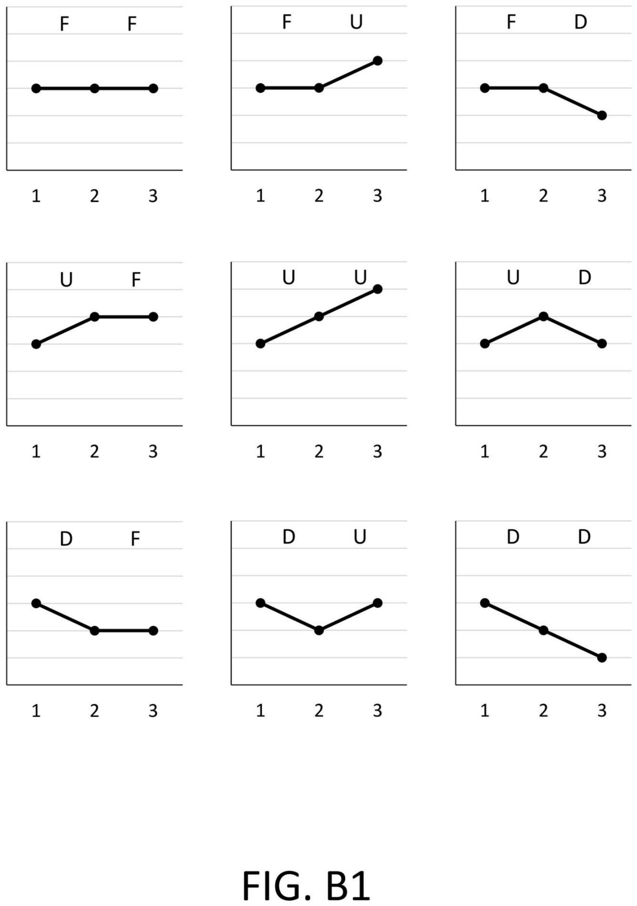

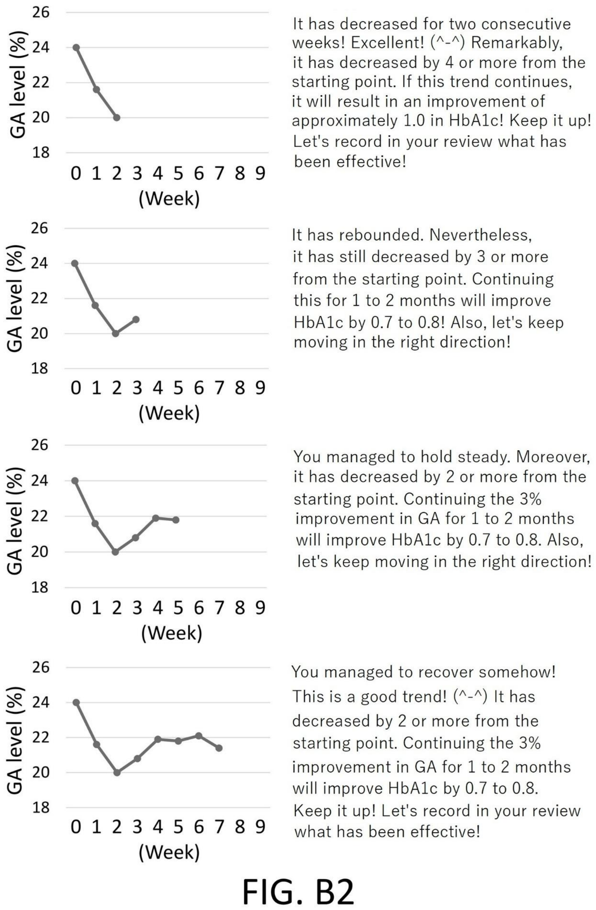

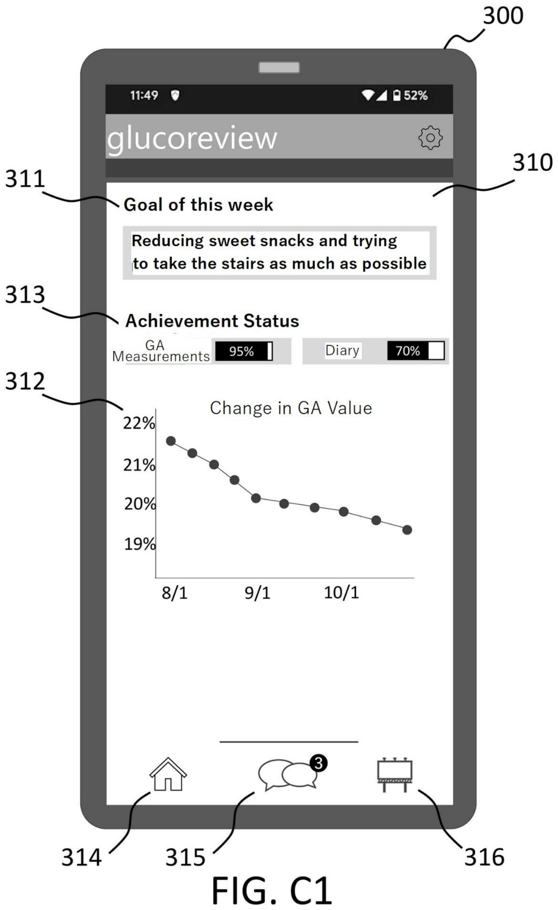

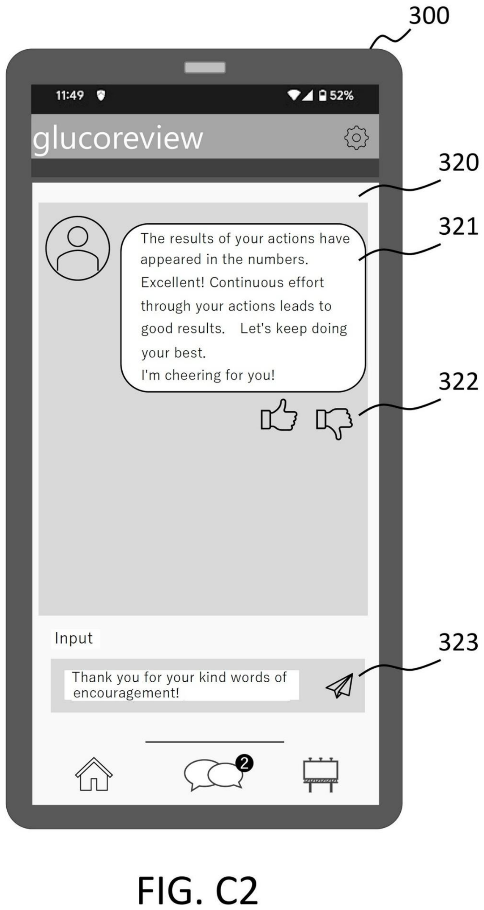

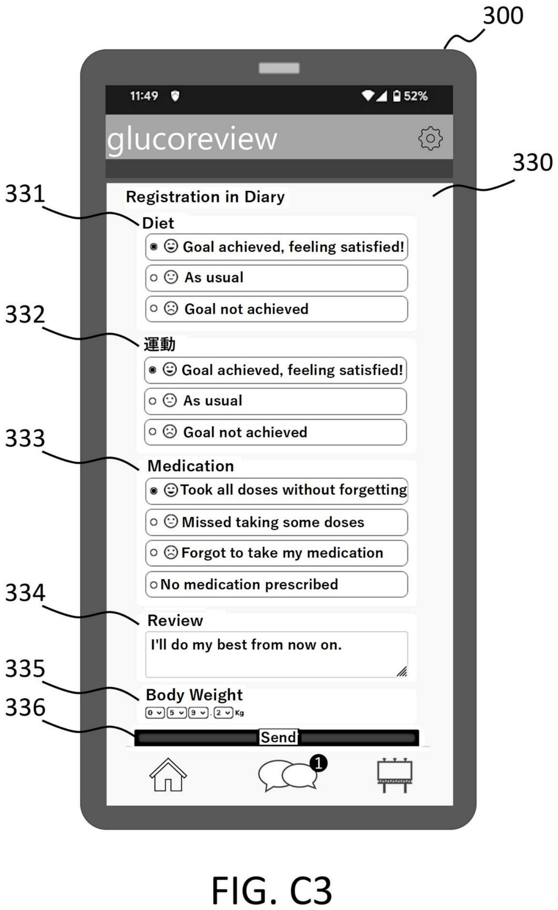

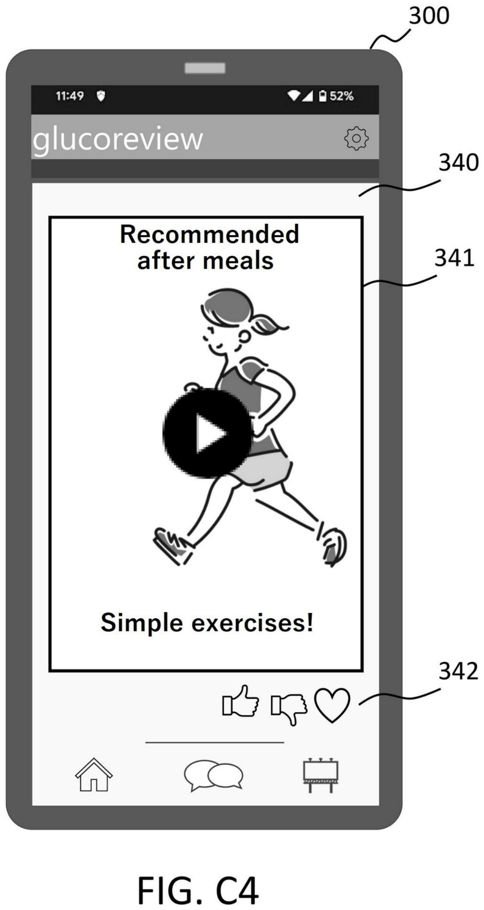

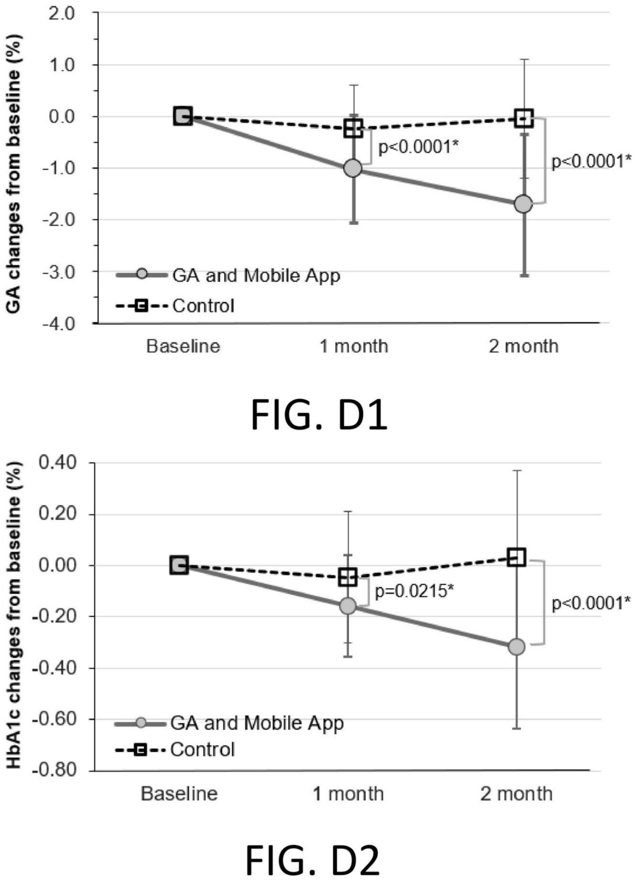

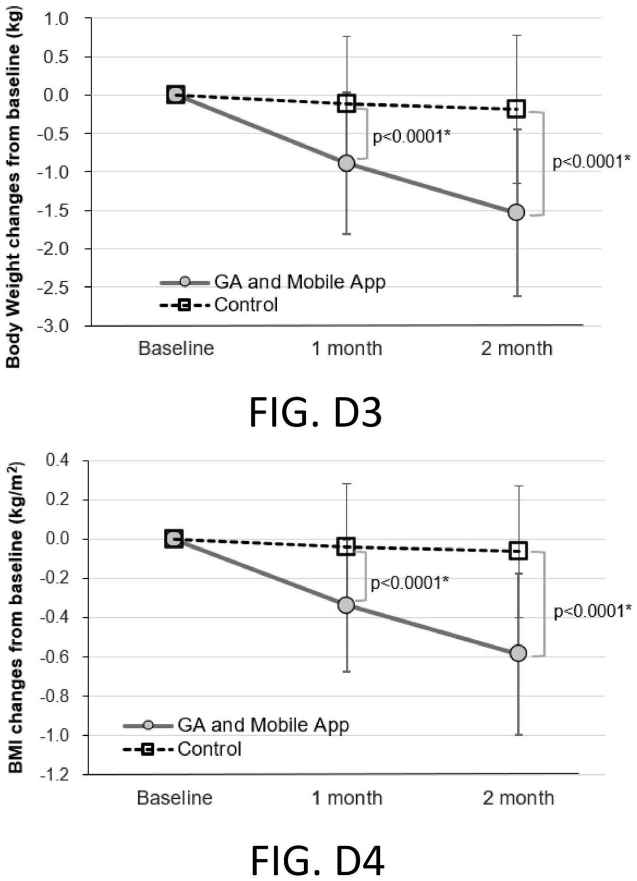

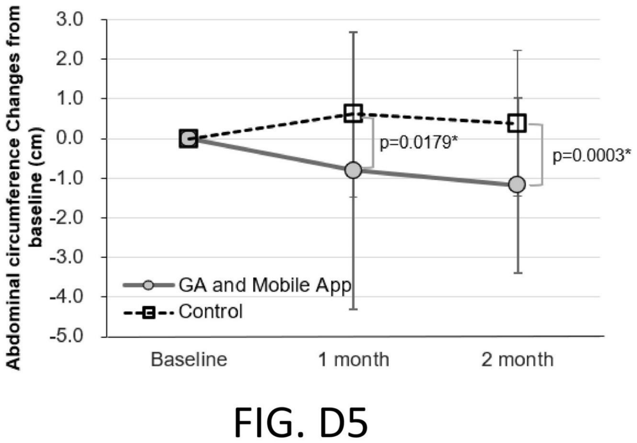

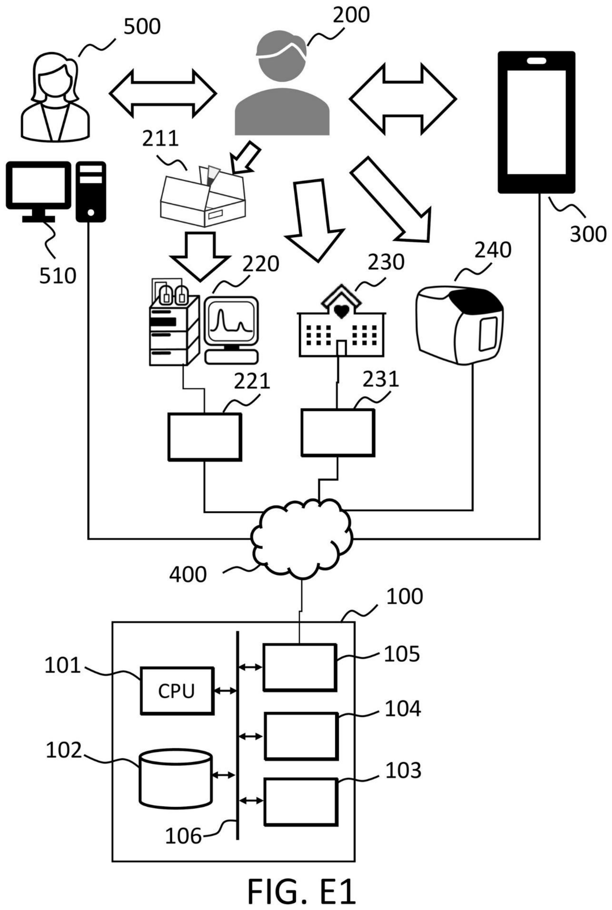

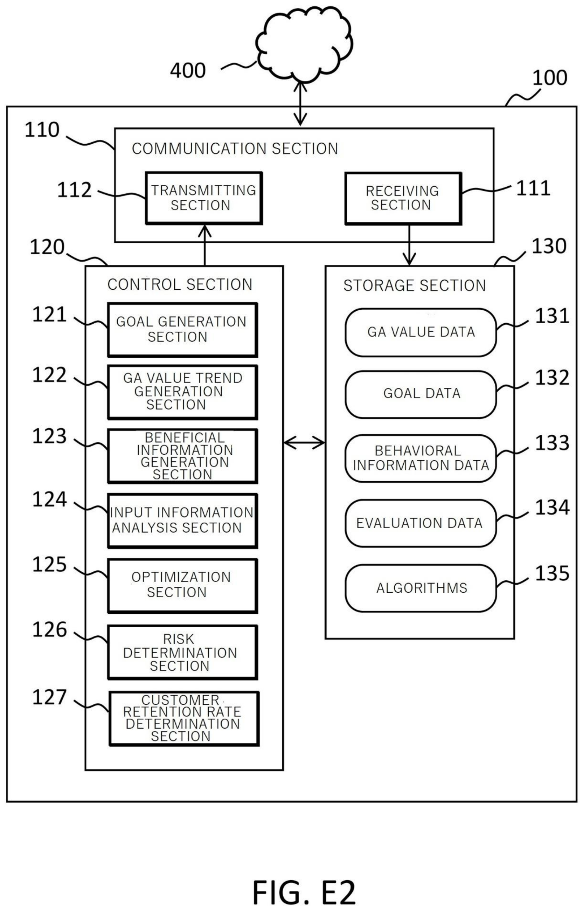

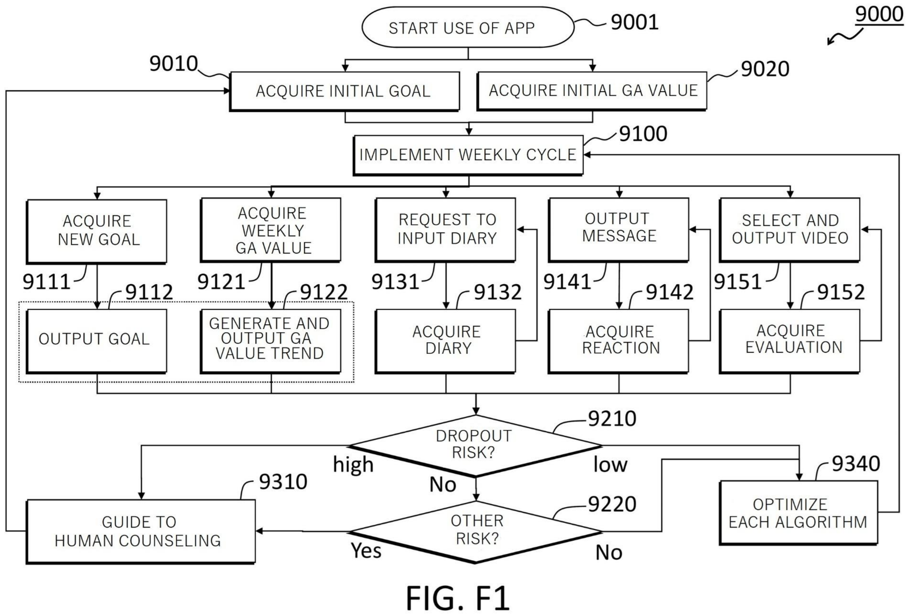

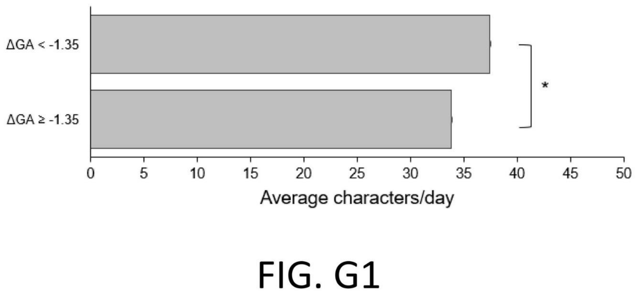

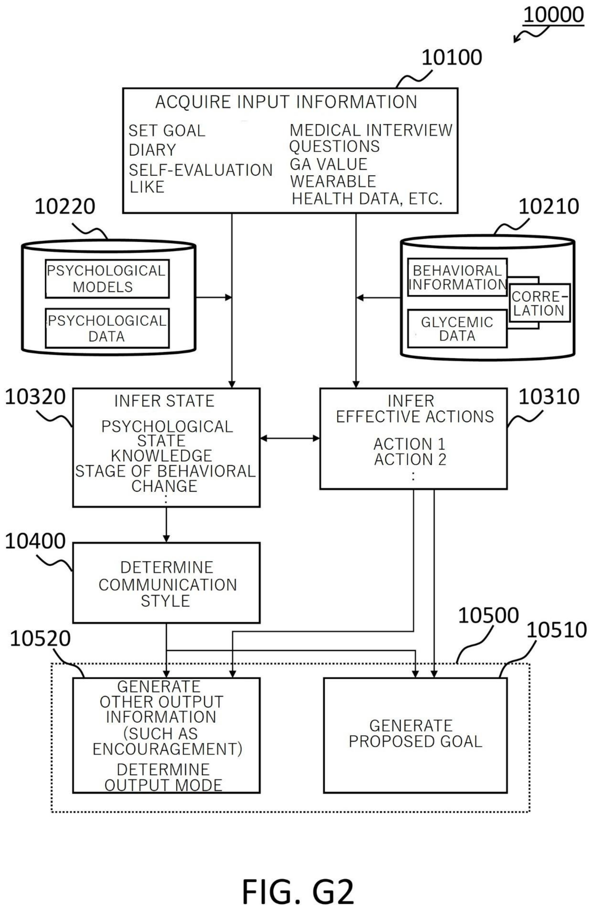

**Claims**

1. A method for assisting glycemic control of a subject using a computer system, the method comprising:

   a) acquiring a self-set goal of the subject;
   b) acquiring a weekly GA value of the subject;
   c) generating a GA value trend using a plurality of the weekly GA values;
   d) outputting the GA value trend to the subject; and
   e) outputting the self-set goal of the subject to the subject.

2. The method according to claim 1, wherein
   the GA value trend and the self-set goal of the subject are output to the subject so as to be recognizable by the subject substantially simultaneously.

3. The method according to claim 1 or 2, further comprising:
   providing an option to the subject to input information related to a behavior of the subject (behavior-related information) to the computer system.

4. The method according to claim 3, wherein

   the goal includes a goal related to diet, exercise, and medication,
   the behavior-related information includes a degree of achievement of the goal of the diet, exercise, and medication, and
   the degree of achievement of the goal of the diet, exercise, and medication is configured to be input as an answer to a multiple-choice question.

5. The method according to claim 3, wherein
   the behavior-related information is configured to be input as a free answer to an open-ended question.

6. The method according to claim 1, wherein

   an input field for the subject to input a user message for a system is provided, and
   the computer system is configured to allow the subject to communicate with the computer system via the user message and a system message.

7. The method according to claim 1, further comprising:

   acquiring behavior-related information of the subject; and
   acquiring a user message of the subject for a system, wherein
   the method comprises:

      generating a system message for the subject, based on at least part of the goal, the GA value trend, the behavior-related information, and the user message; and
      outputting the system message to the subject.

8. The method according to claim 1, further comprising:

   acquiring behavior-related information of the subject; and
   acquiring a user message of the subject for a system, wherein
   the outputting of the goal to the subject comprises generating a goal to be proposed to the subject, based on at least part of the goal, the GA value trend, the behavior-related information, and the user message.

9. The method according to claim 1, further comprising:
   providing educational information to the subject.

10. The method according to claim 9, wherein
    the computer system is configured to allow the subject to input an evaluation regarding the educational information.

11. The method according to claim 1, further comprising:

    acquiring behavior-related information of the subject;
    acquiring a user message of the subject for a system;
    outputting a system message to the subject;
    providing educational information to the subject; and
    acquiring an evaluation by the subject regarding information output or provided to the subject, wherein
    the evaluation by the subject includes at least one of:

    an evaluation regarding the GA value trend;
    an evaluation regarding the educational information; and
    an evaluation regarding the system message, and

    at least one of:

    generating a new goal;
    selecting the educational information; and
    generating the system message

    is performed based on the evaluation by the subject.

12. The method according to claim 11, further comprising:
    based on information input from the subject, optimizing at least one of:

    an algorithm for generating the new goal;
    an algorithm for selecting the educational information; and
    an algorithm for generating the message.

13. The method according to claim 12, wherein
    the optimizing is performed using artificial intelligence.

14. The method according to claim 11, further comprising:

    determining whether the subject has a risk, based on input information input from the subject, wherein
    the risk includes a disease risk and/or a risk of stopping or suspending use of the computer system.

15. The method according to claim 14, further comprising:
    recommending human counseling to the subject when it is determined that the subject has the risk.

16. The method according to claim 12, further comprising:

    acquiring a customer retention rate, wherein
    the optimizing of the algorithm or algorithms includes optimizing the algorithm or algorithms based on the customer retention rate.

17. The method according to claim 1, comprising:

    inferring a state of the subject, based on at least part or all of the self-set goal of the subject, the GA value trend, input information from the subject, and information regarding the subject;
    adjusting a communication style with the subject, based on the state of the subject;
    generating output information, based on the state of the subject and the communication style; and
    outputting the output information to the subject in accordance with the communication style.

18. The method according to claim 17, wherein
the state of the subject includes at least part of a psychological state, knowledge, and a stage of behavioral change of the subject.

19. The method according to claim 17, wherein
the output information includes a goal behavior to be proposed to the subject.

20. A computer system for assisting glycemic control of a subject,
the computer system comprising:

   a processor;
   a communication unit connected to the processor and capable of communicating with an electronic terminal accessible by the subject; and
   a memory connected to the processor, wherein
   the processor is programmed to:

      acquire, via the communication unit, a self-set goal of the subject and store the goal in the memory;
      acquire, via the communication unit, a weekly GA value of the subject and store the weekly GA value in the memory;
      generate a GA value trend with reference to a plurality of the weekly GA values stored in the memory;
      transmit, via the communication unit, the GA value trend to the electronic terminal of the subject and cause the electronic terminal to output the GA value trend to the subject; and
      transmit, via the communication unit, the self-set goal of the subject to the electronic terminal of the subject and cause the electronic terminal to output the self-set goal of the subject to the subject.

21. The computer system according to claim 20, wherein
the GA value trend and the self-set goal of the subject are output to the subject so as to be recognizable by the subject substantially simultaneously.

22. The computer system according to claim 20, wherein
the processor is further programmed to:

   infer a state of the subject, based on at least part or all of the self-set goal of the subject, the GA value trend, input information from the subject, and information regarding the subject;
   determine a communication style with the subject, based on the state of the subject;
   generate output information, based on the state of the subject and the communication style; and
   output the output information to the subject in accordance with the communication style.

23. The computer system according to claim 22, wherein
the state of the subject includes at least part of a psychological state, knowledge, and a stage of behavioral change of the subject.

24. The computer system according to claim 22, wherein
the output information includes a goal behavior to be proposed to the subject.

SUBJECT | SYSTEM

1000

SET INITIAL GOAL → ACQUIRE INITIAL GOAL — 1111

1120

PROPOSE NEXT-WEEK GOAL

AWARENESS BUILDING

SET GOAL → ACQUIRE GOAL — 1112

MEASURE GA VALUE → ACQUIRE GA VALUE — 1210

1220

DETERMINE GA VALUE TREND

RECOGNIZE CHANGE IN GA VALUE

FIG. A1

SUBJECT                                    SYSTEM                              2000

| SET INITIAL GOAL | → | ACQUIRE INITIAL GOAL | 2111 | | PROPOSE NEXT-WEEK GOAL | 2120 |

AWARENESS BUILDING ←

| SET GOAL | → | ACQUIRE GOAL | 2112 |

| MEASURE GA VALUE | → | ACQUIRE GA VALUE | 2210 | | DETERMINE GA VALUE TREND | 2220 |

RECOGNIZE CHANGE IN GA VALUE ←

# FIG. A2

FIG. A3

FIG. A4

FIG. A5

SUBJECT | SYSTEM

6000

SET INITIAL GOAL → ACQUIRE INITIAL GOAL 6111 → ※1

※1 ~ 4 → PROPOSE NEXT-WEEK GOAL 6120

AWARENESS BUILDING ←

SET GOAL → ACQUIRE GOAL 6112 → ※1

※1 ~ 4 → SELECT EDUCATIONAL INFORMATION 6321

IMPROVE UNDERSTAND-ING ←

EVALUATE → ACQUIRE EVALUATION 6331

MEASURE GA VALUE → ACQUIRE GA VALUE 6210 → DETERMINE GA VALUE TREND 6220

RECOGNIZE CHANGE IN GA VALUE ←

EVALUATE → ACQUIRE EVALUATION 6230 → ※2

BEHAVIORAL RECORDS, DIARY, ETC. → ACQUIRE BEHAVIORAL INFORMATION 6410 → ※3

※1 ~ 4 → GENERATE MESSAGE 6322

MOTIVATE ←

EVALUATE → ACQUIRE EVALUATION 6332

ANALYZE INPUT INFORMATION 6520 → ※4

# FIG. A6

46

7000

| SUBJECT | SYSTEM |

SET INITIAL GOAL → ACQUIRE INITIAL GOAL 7111 → ※1

※1~4 → PROPOSE NEXT-WEEK GOAL 7120

AWARENESS BUILDING ←

SET GOAL → ACQUIRE GOAL 7112 → ※1

※1~4 → SELECT EDUCATIONAL INFORMATION 7321

IMPROVE UNDERSTAND-ING ←

EVALUATE → ACQUIRE EVALUATION 7331

MEASURE GA VALUE → ACQUIRE GA VALUE 7210 → DETERMINE GA VALUE TREND 7220

RECOGNIZE CHANGE IN GA VALUE ←

EVALUATE → ACQUIRE EVALUATION 7230 → ※2

BEHAVIORAL RECORDS, DIARY, ETC. → ACQUIRE BEHAVIORAL INFORMATION 7410 → ※3

※1~4 → GENERATE MESSAGE 7322

MOTIVATE ←

EVALUATE → ACQUIRE EVALUATION 7332

ANALYZE INPUT INFORMATION 7520 → ※4

※1~4 → OPTIMIZE EACH ALGORITHM 7620

# FIG. A7

47

8000

| SUBJECT | SYSTEM |

SET INITIAL GOAL → ACQUIRE INITIAL GOAL 8111 →※1

※1~4 → PROPOSE NEXT-WEEK GOAL 8120

AWARENESS BUILDING ←

SET GOAL → ACQUIRE GOAL 8112 →※1

※1~4 → SELECT EDUCATIONAL INFORMATION 8321

IMPROVE UNDERSTAND-ING ←

EVALUATE → ACQUIRE EVALUATION 8331

MEASURE GA VALUE → ACQUIRE GA VALUE 8210 → DETERMINE GA VALUE TREND 8220

RECOGNIZE CHANGE IN GA VALUE ←

EVALUATE → ACQUIRE EVALUATION 8230 →※2

BEHAVIORAL RECORDS, DIARY, ETC. → ACQUIRE BEHAVIORAL INFORMATION 8410 →※3

※1~4 → GENERATE MESSAGE 8322

MOTIVATE ←

EVALUATE → ACQUIRE EVALUATION 8332

ANALYZE INPUT INFORMATION 8520 →※4

DETERMINE RISK 8720

TO INTERVIEW ← INTER-VIEW? 8721

ACQUIRE CUSTOMER RETENTION RATE 8820 → OPTIMIZE EACH ALGORITHM 8620

※1~4

FIG. A8

48

FIG. B1

It has decreased for two consecutive weeks! Excellent! (^-^) Remarkably, it has decreased by 4 or more from the starting point. If this trend continues, it will result in an improvement of approximately 1.0 in HbA1c! Keep it up! Let's record in your review what has been effective!

It has rebounded. Nevertheless, it has still decreased by 3 or more from the starting point. Continuing this for 1 to 2 months will improve HbA1c by 0.7 to 0.8! Also, let's keep moving in the right direction!

You managed to hold steady. Moreover, it has decreased by 2 or more from the starting point. Continuing the 3% improvement in GA for 1 to 2 months will improve HbA1c by 0.7 to 0.8. Also, let's keep moving in the right direction!

You managed to recover somehow! This is a good trend! (^-^) It has decreased by 2 or more from the starting point. Continuing the 3% improvement in GA for 1 to 2 months will improve HbA1c by 0.7 to 0.8. Keep it up! Let's record in your review what has been effective!

# FIG. B2

**FIG. C1**

FIG. C2

FIG. C3

300

340

341

342

FIG. C4

FIG. D1

FIG. D2

FIG. D3

FIG. D4

FIG. D5

FIG. E1

400

100

110 COMMUNICATION SECTION

112 TRANSMITTING SECTION

RECEIVING SECTION 111

120 CONTROL SECTION

130 STORAGE SECTION

121 GOAL GENERATION SECTION

131 GA VALUE DATA

122 GA VALUE TREND GENERATION SECTION

132 GOAL DATA

123 BENEFICIAL INFORMATION GENERATION SECTION

133 BEHAVIORAL INFORMATION DATA

124 INPUT INFORMATION ANALYSIS SECTION

134 EVALUATION DATA

125 OPTIMIZATION SECTION

135 ALGORITHMS

126 RISK DETERMINATION SECTION

127 CUSTOMER RETENTION RATE DETERMINATION SECTION

FIG. E2

FIG. F1

FIG. G1

FIG. G2

## INTERNATIONAL SEARCH REPORT

| | |
|---|---|
| International application No. | |
| | **PCT/JP2024/016833** |

### A. CLASSIFICATION OF SUBJECT MATTER

*G16H 20/00*(2018.01)i
FI:  G16H20/00

According to International Patent Classification (IPC) or to both national classification and IPC

### B. FIELDS SEARCHED

Minimum documentation searched (classification system followed by classification symbols)

G16H20/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2016-48531 A (TERUMO CORP., SYSMEX CORP.) 07 April 2016 (2016-04-07) paragraphs [0023]-[0029], [0044], [0054]-[0056], [0090], [0091], fig. 1, 21 | 1-24 |
| Y | WO 2020/013230 A1 (PROVIGATE INC.) 16 January 2020 (2020-01-16) paragraphs [0024], [0037], [0043], [0044], [0046], [0051], [0054], [0111], [0148] | 1-24 |
| Y | WO 2022/093830 A1 (BECTON, DICKINSON AND COMPANY) 05 May 2022 (2022-05-05) paragraphs [0155], [0193], [0207]-[0213], [0215], fig. 17, 18, 40 | 6-19, 22-24 |

☐ Further documents are listed in the continuation of Box C.      ☑ See patent family annex.

| | | |
|---|---|---|
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 May 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2016-48531 | A | 07 April 2016 | (Family: none) | | | |
| WO | 2020/013230 | A1 | 16 January 2020 | US | 2021/0311070 | A1 | |
| | | | | paragraphs [0043], [0056], [0063], [0064], [0066], [0075]-[0078], [0081], [0144], [0181] | | | |
| | | | | EP | 3822639 | A1 | |
| | | | | CN | 112771378 | A | |
| WO | 2022/093830 | A1 | 05 May 2022 | JP | 2023-548824 | A | |
| | | | | US | 2023/0420090 | A1 | |
| | | | | EP | 4236769 | A1 | |
| | | | | CA | 3198825 | A1 | |
| | | | | AU | 2021370653 | A1 | |
| | | | | CN | 116710924 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- American Diabetes Association, Diagnosis and classification of diabetes mellitus. *Diabetes Care.*, 2014, vol. 37 (1), S81-90, https://doi.org/10.2337/dc14-s081

- **LALLY P** ; **VAN JAARSVELD C** ; **POTTS H** ; **WARDLE J.** How are habits formed: Modelling habit formation in the real world.. *Eur J Soc Psychol.*, 2009, vol. 40 (6), 998-1009